# EUROPEAN PATENT APPLICATION

(11) **EP 4 047 350 A1**
(43) Date of publication of application: **24.08.2022**
(21) Application number: 21157702.8
(22) Date of filing: 17.02.2021
(51) Int. Cl.: G01N 21/31, G01N 33/00, G01N 21/85, G01N 21/3563, G01N 21/71, G01N 21/84, G01N 21/359, G01N 33/02

(54) **METHODS FOR DETERMINING COMPONENTS IN INDUSTRIAL PROCESSING OF SUGAR BEETS IN A PRODUCTION FACILITY**

(71) Applicant: KWS SAAT SE & Co. KGaA, 37574 Einbeck (DE)
(72) Inventor: Hilscher, Elke, 37574 Einbeck (DE); Narten, Heiko, 30966 Hemmingen (DE); Meldau, Stefan, 37077 Göttingen (DE)

(57) **Abstract**

The invention relates to a method for determining components in sugar beets for sugar production and or in animal feed production; in biogas production; in ethanol production; in liquor production, production of biodegradable plastics, fuels or fuel bio-components, food supplement production, betaine, betalaine, betacyanins, betaxanthins, molasses production in a sugar production facility, comprising providing a plurality of sugar beets including a production portion of sugar beets for sugar production and an analysis portion of sugar beets for component analysis and possibly for sugar production, analysing at least the analysis portion and possibly the production portion, wherein analysing comprises emitting electromagnetic waves towards at least the analysis portion and possibly the production portion, receiving electromagnetic waves, converting the received electromagnetic waves into a spectral signal, producing sugar from at least the production portion and possibly from the analysis portion Further, the invention relates to an arrangement for determining components in sugar beets, and to a sugar production facility in animal feed production; in biogas production; in ethanol production; production of biodegradable plastics, fuels or fuel bio-components . Further, the invention relates to a method for generating calibration data for the determination of components in sugar beets for sugar production in animal feed production; in biogas production; in ethanol production; production of biodegradable plastics, fuels or fuel bio-components, and to a use of an analysis assembly and/or an arrangement and/or a method.

## Description

The invention relates to a method for determining components in industrial processing of sugar beets in a production facility, an arrangement for determining components in sugar beets for sugar production, a sugar production facility, and a method for generating calibration data for the determination of components in sugar beets for sugar production. Further, the invention relates to a use of an analysis assembly in a sugar production facility and/or a use of an arrangement for determining components in sugar beets for sugar production and/or a use of a method for determining components in sugar beets for sugar production.

Sugar beets are typically used for sugar production in sugar production facilities. In a sugar production process that consists of several steps, sugar is extracted from the sugar beets. Existing solutions, in particular for extracting sugar from sugar beets, are described for example in US 7,695,566 B2, US 8,328,948 B2, US 8,691,306 B2, US 2020/0208227 A1, and WO 2004/055219 A1.

For determining components in sugar beets and for quality control of the sugar beets received at a sugar production facility, typically a sub-sample of the delivered sugar beets is analysed. Therefore usually, a beet saw, for example as described in DE 2611636 B1, is used with which a fine and homogeneous sugar beet pulp can be prepared from the sugar beets. The sugar beet pulp is extracted using aluminium sulfate or lead acetate solution or water, then analysed by conducting measurements, such as polarimetry, flame photometry, fluorometric o-phthalaldehyde (OPA) method, copper method, and immobilized enzyme biosensor method or others.

Determining components in sugar beets for sugar production and quality control of sugar beets is relatively time consuming as well as labour and cost intensive. Furthermore, only a small fraction of the sugar beets received at a sugar production facility is analysed for quality control. From the sugar beet pulp usually only a sample of 0.3 - 2 wt.%, for example 26 g of sugar beet pulp, is used for determining components. Many process steps from sampling to analysing are necessary and these steps have an influence on the accuracy of the analysis results and are thus only limitedly representative. As a result of such non-representative sampling and analysis, significant distortions and therefore an unreliability may occur in the component analysis, resulting in suboptimal sugar production from the sugar beets.

It is an object of the present invention to provide an improved method for determining components in sugar beets for sugar production, and/or to provide an improved method for generating calibration data for the determination of components in sugar beets for sugar production, and/or to provide an improved arrangement for determining components in sugar beets for sugar production, and/or to provide an improved sugar production facility. In particular, it is an object of the present invention to provide a solution for improving the quality control of sugar beets in a sugar production process and for determining components in sugar beets for sugar production.

According to a first aspect, it is provided a method for determining components in industrial processing of sugar beets in a production facility, comprising providing a plurality of sugar beets including a production portion of sugar beets for production and an analysis portion of sugar beets for component analysis and possibly for production, analysing at least the analysis portion and possibly the production portion, wherein analysing comprises emitting electromagnetic waves towards at least the analysis portion and possibly the production portion, receiving electromagnetic waves, converting the received electromagnetic waves into a spectral signal, producing a product from at least the production portion and possibly from the analysis portion.

Sugar production is preferably conducted in a sugar production facility. In particular, a sugar production facility is understood to be a sugar factory or a sugar beet-processing factory. Preferably, the sugar production facility is adapted to process at least 500 tons of sugar beets per day, or at least 1,000 tons of sugar beets per day, preferably at least 5,000 tons of sugar beets per day, in particular at least 10,000 tons of sugar beets per day. The plurality of sugar beets can comprise several tons of sugar beets. Preferably, the plurality of sugar beets is delivered to the sugar production facility by truck or train. Preferably, the plurality of sugar beets can be provided from a receiving station and/or a storage station.

The plurality of sugar beets comprises a production portion of sugar beets for sugar production and an analysis portion of sugar beets for component analysis. The production portion is preferably at least 99 wt.%, in particular at least 99.9 wt.% or 99.999 wt.%, of the plurality of sugar beets. The production portion can be 100 wt.% of the plurality of sugar beets. In the sugar production process, sugar is extracted from the production portion.

The analysis portion can be a part of the production portion or, at least partly or fully, identical with the production portion. The analysis portion can be a part of the plurality of sugar beets that is not part of the production portion. The analysis portion of sugar beets can also be partly part of the production portion and/or partly part of the plurality of sugar beets that is not part of the production portion. In particular, the analysis portion is, at least primarily, used for component analysis, wherein preferably several components can be determined. Further, also the analysis portion can be used for sugar production. The analysis portion can be chosen randomly or selectively. Preferably, the analysis portion can be selected continuously by a sample provider from the production portion.

The analyses as described herein can be performed on the full analysis portion or on at least a part of the analysis portion. In particular, in case several analyses are performed, each of the analyses can be performed on the whole or a part of the analysis portion. For example, different or overlapping or identical parts of the analysis portion can be used for different analyses. Therefore, any reference herein to the analysis portion can also be understood as a reference to at least a part of the analysis portion.

The components in sugar beets can be one or several of the following group: total sugar content, content of monosaccharides as glucose; fructose; galactose; content of disaccharides as sucrose, lactose, maltose; content of Oligosaccharides as raffinose, maltodextrin, cellodextrin; content of polysaccharides as inulins, fructans; extraction efficiency of sugar; dry matter content, crude protein, crude fiber, amino acids, starch, total sugar content, recoverable sugar content, soluble nitrogen compounds as proteins, betaine, betalaine, amids and amino acids; insoluble nitrogen compounds as unsoluble proteins; nitrogen free organic substances as pectins, saponins, organic acids; marc content; fat content; content of alcohols; phenolic compounds, content of structural carbohydrates as NDF (Neutral Detergent Fiber); ADF (Acid Detergent Fiber); ADL (Acid Detergent Lignin) or content of Hemicellulose; cellulose; Lignin; ash content; content of alkali metal elements and its inorganic compounds as sodium; sodium chloride; content of metal elements und inorganic compounds as calcium; calcium carbonate, magnesium, magnesium oxide; content of metalloid elements and inorganic compounds as boron, borat minerals, selenium, silicon; content of nonmetal elements and inorganic compounds as carbon, carbonates, phosphorus, phosphates; sulfur, iodine.

As will be described in more detail below, the production portion and/or the analysis portion can be provided in sugar beet pieces by chopping and/or slicing. After providing the production portion and/or the analysis portion in sugar beet pieces, the production portion consisting of sugar beet pieces is still referred to as production portion and the analysis portion consisting of sugar beet pieces is still referred to as analysis portion. The analysis portion and the production portion can be chopped and/or sliced into sugar beet pieces using the same device, e.g. a slicing device, or different devices, e.g. a slicing device and a chopping device.

Preferably, the electromagnetic waves that are emitted towards at least the analysis portion and possibly the production portion lie within the infrared spectrum, in particular in the near-infrared spectrum and/or in the visible spectrum and/or in the ultraviolet spectrum. Preferably, the electromagnetic waves have at least one wavelength, wherein the wavelength of the electromagnetic waves lies in the range of 170 nm to 1,000,000 nm, in particular in the range of 750 nm to 2,500 nm, preferably in the range of 780 nm to 1800 nm. Preferably, the spectral signal is converted by using spectroscopy, in particular near-infrared spectroscopy (NIRS), and/or mid-infrared- spectroscopy, and/or far-infrared- spectroscopy and/or ultraviolet-visible spectroscopy (UV-Vis) and/or Raman spectroscopy and/or laser-induced breakdown spectroscopy (LIBS).

Preferably, the electromagnetic waves are reflected from at least the analysis portion that is arranged on the transport device and the reflected electromagnetic waves are received, in particular with a sensor that is adapted to receive electromagnetic waves. Preferably, the electromagnetic waves are reflected continuously from the analysis portion and the production portion. Preferably, the received electromagnetic waves are reflected by the analysis portion and possibly the production portion.

Preferably, the received electromagnetic waves are converted into a spectral signal, wherein the spectral signal is generated dependent on the received electromagnetic waves.

Preferably, producing sugar comprises producing a raw juice from at least the production portion and possibly from the analysis portion, comprising extracting sugar from at least the production portion in an extraction process, wherein at least the production portion is arranged in hot water and the hot water flows in opposite direction to a direction in which at least the production portion is transported, wherein sugar is removed from at least the production portion into the hot water and the raw juice is made from the hot water containing the extracted sugar. Preferably, the raw juice contains about 98 wt.% of the sugar contained in the production portion and organic and inorganic constituents that can be referred to as non-sugars from the sugar beets.

The method steps can be carried out in an order that deviates from the listed order. However, it is preferred if the method steps are carried out in the listed order, in particular one after the other.

The solution described herein has the advantage that analyzing sugar beets in a sugar production process can be automated by using spectral signals. The solution results in a determination of components in sugar beets that is less labour and cost intensive as well as less time consuming. Determining components in sugar beets can be done simultaneously with the sugar production process. It is particularly advantageous that components can be determined without delay. With a solution described herein a real-time determination of components can be achieved.

Furthermore, the solution described herein has the advantage that the sugar production process can be improved, and in particular made more efficient, because from the information of the spectral signals sugar production parameters can be adjusted, dependent on the components of the sugar beet pieces that are currently in the sugar production process or that are introduced into the sugar production process. With the information derived from the spectral signals, improved (and real-time) analysis data can be available, with which it is possible to adjust sugar production parameters more accurately for an improved sugar production process.

Typically, the extraction of sucrose from beet pulp is one of the most important processes of a sugar production process. It can for example determine the loss of sucrose in sugar beet pieces, molasses, energy cost and raw juice quality. Usually for determining components in sugar beets for sugar production and for sugar beets processed at a sugar production facility, typically a small amount of sliced beets and/or dried pulp, particular on sugar beet pieces in form of slices are analysed. The slices and/or dried pulp are mashed to a homogeneous sugar beet pulp by a cutter mill. The sugar beet pulp is extracted by Aluminium sulfate or lead acetate solution or water, and then analysed by conducting measurements, such as polarimetry, flame photometry, fluorometric o-phthalaldehyde (OPA) method, copper method, and immobilized enzyme biosensor method or others. The slices of fresh sugar beets and/or dried pulp can be analysed for sugar content, dry matter, marc content to evaluate the right extraction conditions (temperature, time, pressure). Raw juice can comprise beside sugar non-sugars such as water, pectin, proteins, and pigments and other components. Raw juice sugar is preferably further purified. Preferably, an important stage of sugar production can be refining of raw juice, wherein juice purity can directly be related to removing non-sugar material. The extraction process itself can have a great effect on the composition of the raw juice. The purity of clarified juice can be higher than that of the raw juice, an increase in purity and a corresponding decrease in non-sugars in clarified juice can indicate that during clarification process it has been ensured that the non-sugars are removed or greatly decreased when the precipitation and absorbing agents are used. Preferably, the evaporation and crystallization processes can reduce the concentration of non-sugars and increase the purity of syrup and molasses. Preferably, the knowledge about all sugar beet quality parameters including sugars and all non-sugar components allows to ensure the adjustment of all processing and juice purification steps in sugar production facility.

Therefore, a further advantage of the solution described herein is that a method is developed for a real-time quality testing for determining components in sugar beets for sugar production, an arrangement for determining components in sugar beets for sugar production, and a sugar production facility. Further, the invention allows for the use of an application of Process Analytical Technology (PAT) equipment in a sugar production facility and/or an arrangement from off-line to real-time quality testing for determining components in sugar beets for sugar production in a sugar production facility and/or a method for determining components in sugar beets for sugar production in a sugar production facility. With the solution described herein, new quality aspects of sugar beets can be determined, and production processes can be adjusted and/or regulated to increase the process efficiency in a sugar production facility.

A further advantage is that a significantly larger quantity of the sugar beets that are received at a sugar production facility can be analysed and that determining components in sugar beets for sugar beet production can be conducted on a significantly larger amount of sugar beets compared to the use of existing analysis methods used for analysing sugar beets that are determined for sugar production, for example for quality control.

The solution described herein overcomes the disadvantages of existing solutions, where only a very small amount of the sugar beets is analysed and determining components in sugar beets for sugar production is only conducted on a very small amount of the sugar beets in a sugar beet production process.

A further advantage is that in the solution described herein all parts of the sugar beets can be analysed, as the sugar beets are chopped into sugar beet pieces before component analysis. This can lead to a higher accuracy of determining components, when compared to component analysis by analysing sugar beet pulp, as the concentration and the content of components in sugar beets are irregularly distributed within a single sugar beet and between different sugar beets.

A further advantage of the solution described herein lies in the fact that in sugar production facilities typically only polarization and/or sugar content of the sugar beets is analysed. This information is used to estimate the quality of the sugar beets and farmers receive a payment dependent on the estimated quality. However, these analyses are based on very few parameters, such as Sodium, Potassium, alpha-amino-N, sugar content, and do not sufficiently reflect the chemical complexity of a sugar beet and how well the sugar can be extracted from the sugar beet. With the solution described herein, an entire range of organic compounds in sugar beets can be detected. Thus, with the solution described herein it is possible to estimate the quality of sugar beets more accurately beyond the classical analysis.

A further advantage of the solution described herein lies in the fact that problems in analysing sugar beets that are caused by the fact that sugar beets are composed heterogeneously in respect to their components can be overcome. In particular the structure and composition of samples that are used for analysis can be crucial for the accuracy of determining components, such as quality parameters. Genomic parameters, crop cultivation, and environmental conditions can influence growth and content of components in sugar beets. For example, the concentration and the content of the components in sugar beets, such as for example the distribution of sucrose, dry matter, α-amino-nitrogen, soluble nitrogen, total nitrogen, glucose, fructose, sodium and potassium, within sugar beets can be irregular distributed within a sugar beet and between different sugar beets. A classical component analysis for determining components can usually be done in a laboratory, wherein a representative beet pulp can be produced. For more than 60 years this has been done using a beet saw which can supply fine and homogeneous sugar beet pulp that is required in sufficient quantity and quality for the sugar beet analysis. After production and storage, this sugar beet pulp can be converted into an aqueous filtrate by cold aqueous digestion with aluminum sulfate (ICUMSA GS 6-3) as clarifying agent for subsequent analysis. The International Commission for Uniform Methods of Sugar Analysis (ICUMSA) is an international standards body that publishes detailed laboratory procedures for the analysis of sugar. The procedures have specific steps involved. The quality parameters can be analysed by an automatic beet laboratory, sucrose can be analysed by using a polarimeter (ICUMSA GS 6-3), Potassium and Sodium by using a Flame- Photometer (ICUMSA GS 6-7) and α-amino-N by using fluorometric OPA method or by the copper method ('Blue Number') (ICUMSA GS6-5). The concentration of Glucose can be determined by an immobilized Enzyme Biosensor Method (ICUMSA GS6-8). Within such a classical component analysis, from the whole produced pulp usually only a sample, for example of about 0.3 - 2 wt.%, can be used for the component analysis itself. Many process steps from sampling to analysing are typically necessary and can have an influence on the accuracy of the results of the component analysis that may be only limitedly representative. As a result of a non-representative sampling, significant distortions may occur in the measurement of ingredients. However, applying the solution described herein can reduce the sample error and therefore the prediction error for the entire sample. The solution described herein overcomes these shortcomings pointed out above in that a much larger sample and/or a much larger amount of sugar beets can be analysed. This can advantageously result in a reduction of the sample error and therefore a reduction of the prediction error for the analysed sugar beets.

The success in production of sugar from sugar beets can not only be affected by the total sugar content in sugar beets but also by components which interfere with sugar the manufacturing process, such as non-sugar components of the beets. For example, the influence of the non-sugar components on sugar extractability has been subject to several investigations, wherein different formulas were developed. For example, Buchholz et al. (Buchholz, K.; Märländer, B.; Puke, H.; Glattkowski, H.: Neubewertung des technischen Wertes von Zuckerrüben. Zuckerind.120(1995)113-121) has established a formula for quality estimation of sugar beets based on 60 sugar beet varieties and molasses analysis. The consistent way of obtaining an indirect determination of the amount of sugar molasses from semi-technical molasses using betaine content of sugar beets and molasses as a reference value, required extensive processing of the sugar beets in a pilot plane (Bruhns, M.; Sievers, C.; Bliesener, K.-M.; Miehe, D.: Neue Technikumsanlage zur Rübenverabeitung am Zuckerinstitut Braunschweig. Zuckerind. 118 (1993)450-454). Due to the use of variables such as Sodium, Potassium and amino-nitrogen this equation can be applied in quality estimation of sugar beets and payment of the farmers. In order to realistic evaluate sugar beet according to their quality, it is preferred if also the acid formation from the degradation of reducing sugars (invert sugars) is taken into consideration. A further problem with the known formula can be that during sugar processing the soluble nitrogen compounds reaching the thick juice (the so-called harmful nitrogen) and detection of all α-amino-nitrogen compounds (analysed in sugar beets) can make up only 35-40% total harmful nitrogen. Due to the importance of harmful nitrogen in sugar processing and quality evaluation of sugar beet it has become essential to consider these compounds in factory evaluation. Formulas were developed by Burba and Schiweck to predict the alkanity reserves in sugar beets (Burba, M.; Schiweck,H.: Nichtzuckerbilanz und Ionenbilanz im Dicksaft als Grundlage einer Qualitatsbewertung von Zuckerrüben. Zuckerind. 118(1993) 680-689 and 924-936). Formula of Burba and Schiweck required the analysis of invert sugar as an additional analytical parameter. All of these formulas lack an analytical method for total nitrogen in thick juice. Until now, a common way to analyse the harmful nitrogen is done by measuring α-amino-N, due to the fact that there is a high correlation to the total harmful soluble nitrogen. It is of high importance to include a high nitrogen supplier such as betaine in beet quality calculation, but until now there are no fast-analytical approaches available to achieve this.

Beside Nitrogen content a lot of scientific efforts have been made to determine the various quality parameters of sugar beet and in the technical juices extracted from them as shown in the following table (Oltmann, W.; Burba. M; Bolz, G.: Die Qualitat der Zuckerrübe. Bedeutung, Beurteilungskriterien und Maßnahmen zu ihrer Verbesserung. Berlin und Hamburg 1984).

| Parameter | | |
|---|---|---|
| Biological | Outer condition | Root tape- top tare; dirt; trash; relative amount of beet tops; relative amount of beet tails |
| | Bolting resistance | |
| | Resistance to diseases | |
| | Variety | Environmental stability |
| | Storability | Sucrose losses by respiration |
| Chemical | Sugar content | |
| | Marc content | |
| | Non-sugar components | |
| | Harmful soluble nitrogen | Glutamine; betaine; pyrrolidone; Asparagine |
| | | |
| | Invert sugar | |
| | Pectins | |
| | Dextran | |
| | Raffinose | |
| Physical (mechanical) | External influences | Crushing, breakage; woody and fibrous beets, elasticity; compressibility |

Until now it has not been possible to arrive to a commonly accepted, binding solution to the problem, explaining importance of each parameter and expressing them in a single formula or a single term (Burba, M.: Perspectives and Limits of current beet quality evaluation. Zuckerind. 123(1998)5, 365-374).

It is an advantage of the solution described herein that these shortcomings can be overcome. Light of specific wavelengths, such as for example in the near-infrared range, can be absorbed by molecules containing C-H; N-H; S-H; O-H groups (fat; carbohydrates; organic acids; structural carbohydrates; water, alcohols, phenolics). The information present in a spectral signal can be used to estimate the concentration of a given substance in a sample or to estimate a bulk or physical property when these can be reflected and result in significant changes in the intensity and/or the wavelength of the spectral signals that is obtained from reflected electromagnetic waves.

A further advantage of the solution described herein lies in the fact that the payment of farmers can be done dependent on the sugar beet quality. Typically, in sugar production facilities only polarisation and/or recoverable sugar content are analysed based on the so called Braunschweiger formula to estimate the quality of sugar beets and to calculate a payment for the farmers based on these calculations. However, these calculations are based on very few parameters (Na, K, alpha-amino-N, sugar) and do not sufficiently reflect the chemical complexity of a sugar beets and how well the sugar can be extracted out of the sugar beets. With the solution described herein, it can be possible to detect an entire range of organic compounds in sugar beets that can be used to estimate the quality of sugar beets beyond the classical analysis. Using mathematical models, such as neuronal networks, can help to identify specific features of spectral signals that can correlate well with the extraction efficiency of the sugar beets. This information can be used to assess the quality of a delivery by a farmer much better than the classical analysis of only a few parameters and can then be used for optimizing the payment system of farmers. In this way the sugar beet-processing factory can benefit from better distinguishing high-quality sugar beets from low quality sugar beets and the farmers get incentivized to deliver best quality for the extraction efficiency of sugar out of sugar beets.

A further advantage of the solution described herein lies in the fact that information of sugar beet quality obtained from component analysis of sugar beets at a receiving section in a sugar production factory can be combined with information from which field or fields these sugar beets were harvested and how this field or these fields were agronomically managed. With such a combination of information growing conditions of sugar beets can be increased, which can lead to an increased quality of sugar beets for future sugar beet deliveries. It is a further advantage that farmers can get more information about components in sugar beets and on growing conditions that can lead to a higher quality of sugar beets for sugar extraction. For example, the information regarding the sugar beet quality can be exchanged with farmers, and/or farmers can exchange information about growing conditions and agricultural practices (e.g. obtained, collected and analysed using farm-management software) with the sugar production facility and/or other third parties, such as farm-management software providers or seed producers or retailers.

According to a preferred embodiment, the production facility is a production facility for sugar production and/or for production of animal feed and/or for biogas production and/or for ethanol production and/or for production of biodegradable plastics and/or for production of fuels and/or for production of fuel bio-components, and/or wherein the production is sugar production and/or production of animal feed and/or biogas production and/or ethanol production and/or production of biodegradable plastics and/or production of fuels and/or production of fuel bio-components, and/or wherein the product is sugar and/or animal feed and/or biogas and/or ethanol and/or biodegradable plastics and/or fuels and/or fuel bio-components.

Preferably, in industrial processing of sugar beets sucrose is extracted from sugar beets using hot water, resulting in raw juice, which can then be purified, filtered, and concentrated by cyclic rinsing and evaporation. To obtain the final product, the thick juice can be crystallized, which can result in white sugar that can then recrystallized, which ultimately can lead to the production of high-quality refined sugar. Various sugar beet products can be produced at different stages of sugar beet processing. A byproduct, which can contain a large amount of water, can comprise up to 75% of beet pulp. The beet pulp can be used as a heat source and, circulating in a closed system, can be used repeatedly to provide a large proportion of heat demands of a sugar production facility. Other by-products can be components that are used as supplements in food production or the pharmaceutical industry, including betaine, betalain, betacyanins, betaxanthins. Following the extraction of sucrose, the sugar beet pulp and beet splinters are preferably used in animal feed production and/or biogas production. Sugar beet leaves can also be used for production of methanol. After centrifugation of thick syrup in a sugar production process, the molasses obtained can be used for the production of alcohol, for a production of animal feed, and/or as a medium for yeast biomass production. Raw sugar beet juice can be used as feedstock for ethanol fermentation. Ozonation can be used to stabilize new kinds of fermentation media used in the biotechnological production of liquid fuel additives. Ethanol obtained in this way can be relatively inexpensive and can be used as a fuel or fuel additive. Hydrolysates of sucrose can be used as raw materials for the production of biodegradable plastics, fuels and/or fuel bio-components.

According to a preferred embodiment, the method further comprises receiving the plurality of sugar beets including the production portion of sugar beets for sugar production and the analysis portion of sugar beets for component analysis and possibly for sugar production, and/or providing sugar beet pieces from the production portion and/or the analysis portion, and/or arranging at least the analysis portion and possibly the production portion onto at least one transport device, and/or conveying at least the analysis portion and possibly the production portion using the at least one transport device, preferably with a transport velocity within a range of 0.05 m/s to 20 m/s, in particular 0.05 m/s to 10 m/s, preferably within a range of 0.05 m/s to 5 m/s, more preferably within a range of 0.05 m/s to 1 m/s.

Preferably, the method comprises producing pressed pulp by removing liquid substances from at least the production portion and/or at least the analysis portion, preferably using a mechanical press.

Preferably, receiving the plurality of sugar beets is conducted by receiving a truckload of sugar beets and/or a container of sugar beets and/or a goods wagon of sugar beets at the sugar production facility, in particular at a receiving section of the sugar production facility.

The method comprises arranging at least the analysis portion onto at least one transport device. Preferably, the method comprises arranging the analysis portion and the production portion onto at least one transport device. The at least one transport device is understood to be a device for conveying sugar beets and/or sugar beet pieces, in particular the analysis portion. The at least one transport device can be adapted to convey the analysis portion and the production portion. Preferably, the at least one transport device comprises or is a conveyor belt.

Preferably conveying at least the analysis portion using the at least one transport device is conducted along a transport direction, wherein a stream of at least the analysis portion is generated. Preferably, the stream of at least the analysis portion moves along the transport direction. Preferably, the method comprises conveying the analysis portion and the production portion using the at least one transport device. The at least one transport device can be arranged at a receiving station, in particular at a position where sugar beets are received. The at least one transport device can be arranged next to and/or at a storage station that is adapted to store sugar beets. The at least one transport device can be arranged next to another transport device in a sugar production facility, in particular next to a transport device that is adapted to convey sugar beets and/or sugar beet pieces. The at least one transport device can comprise several transport sub-devices, in particular transport sub-devices that are arranged next to each other, wherein at least a part of the transport sub-devices are arranged in a sugar production facility. The at least one transport device can be adapted to convey sugar beets and/or sugar beet pieces.

Preferably, producing sugar comprises producing a raw juice from at least the production portion, and/or producing a thin juice, preferably from at least the raw juice, and/or producing a thick juice, preferably from at least the thin juice, and/or producing sugar, preferably from at least the thick juice.

Preferably, the thin juice is produced from the raw juice by cleaning the raw juice and subsequent filtration of the cleaned juice, wherein cleaning the raw juice comprises extracting and removing non-sugars using lime and carbonic acid gas. Preferably, filtration of the cleaned juice comprises filtering out flocculatable insoluble non-sugars and lime.

Preferably, the thick juice is produced by thickening the thin juice in a, preferably multi-stage, evaporation process, comprising evaporating liquid from the thin juice. In such a way, thick juice can be made from the thin juice.

Preferably, sugar is produced from the thick juice by the following steps: further thickening the thick juice, in particular under vacuum conditions, wherein a crystallization process of sugar crystals occurs, centrifugation of the further thickened thick juice, wherein sugar crystals are separated from syrup due to centrifugal forces. Preferably, the produced sugar contains at least 99.7 % sucrose. Subsequently, the produced sugar can be dried, in particular by using an air stream. By drying the sugar, the sugar content can be increased and the moisture content can be decreased.

According to a further preferred embodiment of the method, analysing at least the analysis portion is conducted after the step of receiving a plurality of sugar beets, and preferably prior to storing the plurality of sugar beets. Preferably, the analysis portion is crumbled and/or cut into sugar beet pieces prior to analysing at least the analysis portion. Preferably slicing and/or cutting the production portion into sugar beet pieces is conducted after analysing at least the analysis portion. Analysing at least the analysis portion can be conducted at a receiving station, wherein preferably at the receiving station sugar beets are received. Preferably, when analysing at least the analysis portion is conducted after the step of receiving a plurality of sugar beets and preferably prior to storing the plurality of sugar beets, the transport velocity lies within a range of 0.05 m/s to 20 m/s, in particular 0.05 m/s to 10 m/s, preferably within a range of 0.05 m/s to 5 m/s, more preferably within a range of 0.05 m/s to 1 m/s.

According to a further preferred embodiment of the method, analysing at least the analysis portion is conducted after and/or during storing the plurality of sugar beets, and preferably prior to providing sugar beet pieces, in particular by slicing, Preferably, slicing and/or cutting the production portion into sugar beet pieces is conducted after analysing at least the analysis portion. Analysing at least the analysis portion can be conducted at a storing station, wherein preferably at the storing station sugar beets are stored. Preferably, when analysing at least the analysis portion is conducted after and/or during storing the plurality of sugar beets, and preferably prior to providing sugar beet pieces, in particular by slicing, the transport velocity lies within a range of 0.05 m/s to 20 m/s, in particular 0.05 m/s to 10 m/s, preferably within a range of 0.05 m/s to 5 m/s, more preferably within a range of 0.05 m/s to 1 m/s.

According to a further preferred embodiment of the method, analysing at least the analysis portion and possibly the production portion is conducted after providing sugar beet pieces, in particular by slicing, and preferably prior to producing a raw juice from at least the production portion, Preferably the analysis portion is sliced and/or cut into sugar beet pieces prior to analysing at least the analysis portion. Analysing at least the analysis portion can be conducted within a sugar production process, in particular in a sugar production facility. Analysing at least the analysis portion can preferably be conducted on sliced and/or cut sugar beet pieces within a sugar production facility. Preferably, when analysing at least the analysis portion and possibly the production portion is conducted after providing sugar beet pieces the transport velocity lies within a range of 0.05 m/s to 20 m/s, in particular 0.05 m/s to 10 m/s, preferably within a range of 0.05 m/s to 5 m/s, more preferably within a range of 0.05 m/s to 1 m/s. It is particularly advantageous that analysing can be conducted without homogeneously distributing sugar beet pieces on the at least one transport device. Preferably, at least the analysis portion can be analysed during the sugar beet pieces are conveyed and without the need to further arrange the sugar beet pieces on the at least one transport device.

According to a further preferred embodiment of the method, analysing at least the analysis portion and possibly the production portion is conducted after producing pressed pulp, preferably prior to and/or after drying of the pressed pulp. Preferably the analysis portion comprises and/or is pressed pulp. Preferably the analysis portion is sliced and/or cut into sugar beet pieces prior to analysing at least the analysis portion. Preferably, when analysing at least the analysis portion and possibly the production portion is conducted after producing pressed pulp the transport velocity lies within a range of 0.05 m/s to 20 m/s, in particular 0.05 m/s to 10 m/s, preferably within a range of 0.05 m/s to 5 m/s, more preferably within a range of 0.05 m/s to 1 m/s. Preferably pressed pulp is produced by removing liquid substances from at least the production portion and/or at least the analysis portion, preferably using a mechanical press, wherein the pressed pulp comprises the analysis portion, and/or emitting electromagnetic waves towards the analysis portion, and/or receiving, in particular reflected, electromagnetic waves that were reflected from the analysis portion, wherein preferably the wavelength of the electromagnetic waves lies in the infrared spectrum, in particular in the near-infrared spectrum and/or in the visible spectrum, and/or converting the reflected electromagnetic waves into a spectral signal. Preferably, after sugar has been extracted from at least the production portion in a sugar production process, the sugar beet pieces from which the sugar has been extracted are mechanically pressed, in particular using the mechanical press with which liquid in the sugar beet pieces can be extracted. Preferably, at least 20 %, in particular at least 44 %, of the volumetric and/or gravimetric moisture content of the sugar beet pieces is extracted by using the mechanical press and/or by removing liquid substances from at least the production portion. The volumetric moisture content can be understood as the ratio of the volume of water to the total volume of the material. The gravimetric moisture content can be understood as the ratio of the mass of water to the total mass of the material. Preferably, the extracted liquid is reintroduced into the sugar production process, as the extracted liquid contains sugar. Subsequently, the pressed pulp can be dried. To determine components in the pressed pulp, the method described herein can be applied. From the spectral signal, component analysis can be carried out. In particular, from the spectral signal volumetric and/or gravimetric moisture content and/or sugar content in the pressed pulp can be determined.

According to a further preferred embodiment of the method, analysing is performed as a continuous process, preferably by emitting electromagnetic waves and/or receiving electromagnetic waves and/or converting the received electromagnetic waves into a spectral signal in intervals of less than 100 ms, in particular of less than 50 ms, preferably in intervals of 1 ms, 10ms, 20 ms, 30 ms, or 40 ms. The intervals can also be 1 s, 10 s, 20 s, 30 s or 40 s or several minutes or several hours

Preferably, the intervals between generating spectral signals are less than 100 ms, in particular less than 50 ms, preferably 10 ms, 20 ms, 30 ms or 40 ms. Preferably the intervals between converting received electromagnetic waves into spectral signals are less than 100 ms, in particular less than 50 ms, preferably 10 ms, 20 ms, 30 ms or 40 ms.

According to a further preferred embodiment of the method, the mass fraction of the analysis portion is at least 0.001 % or at least 0.1 % or at least 0.2 % or at least 0.5 % or at least 1 % or at least 10 % or at least 25, or at least 50 %, or at least 80 % within the plurality of sugar beets.

Preferably, the production portion comprises the analysis portion. Preferably, the production portion and the analysis portion are, at least partly, identical. The production portion and the analysis portion can comprise the same sugar beets and/or sugar beet pieces. The production portion and the analysis portion can consist of the same sugar beets and/or sugar beet pieces.

An advantage of a mass fraction of the analysis portion of at least 0.001 % lies in the fact that in particular in sugar production processes usually much smaller mass fractions of the analysis portion are investigated. Therefore, a much higher mass fraction of analysed sugar beets and/or sugar beet pieces can be achieved.

It is possible that the mass fraction of the analysis portion is a large fraction within the plurality of sugar beets, such as for example a mass fraction of 50 % or 80 % within the plurality of sugar beets. The mass fraction of the analysis portion can be 100 % within the plurality of sugar beets. In this case, component analysis is carried out on all sugar beets of the plurality of sugar beets. The volume fraction of the analysis portion can be at least 0.001 % or at least 0.1 % or at least 0.2 % or at least 0.5 % or at least 1 % or at least 10 % within the plurality of sugar beets. The volume fraction of the analysis portion can be at least 50 %, in particular 100 %, within the plurality of sugar beets. The mass fraction of the analysis portion within the plurality of sugar beets can be understood as the ratio of the mass of the analysis portion to the total mass of the plurality of sugar beets, wherein the mass fraction can be expressed as percentage by mass. The volume fraction of the analysis portion within the plurality of sugar beets can be understood as the ratio of the volume of the analysis portion to the total volume of the plurality of sugar beets, wherein the volume fraction can be expressed as volume percent.

According to a preferred embodiment of the method, the production portion comprises the analysis portion. It is preferred if at least a part of the production portion is the analysis portion. In this case, at least a part of the sugar beets and at least a part of the sugar beet pieces that belong to the production portion and that are used for sugar production in a sugar production facility are used for component analysis. This is particularly advantageous as the sugar beets on which component analysis is carried out can also be used for sugar production. Therefore, less or no sugar beets for component analysis need to be discarded and thus, the efficiency of sugar production can be increased.

According to a preferred embodiment of the method, the method comprises generating of calibration data, including taking a sample of sugar beets, preferably a determined amount, for example 10kg, and preferably moving the sample along a sensor that is adapted to receive electromagnetic waves, spectroscopic analysis of the sample by, preferably continuously, emitting electromagnetic waves towards the sample, receiving electromagnetic waves, and converting the received electromagnetic waves into a spectral signal, producing a sugar beet pulp from the sample, preferably by a cutter mill, and extracting the sugar beet pulp, preferably by aluminium sulfate or lead acetate solution or water, reference analysis of the extracted sugar beet pulp by conducting measurements, such as polarimetry, flame photometry, fluorometric o-phthalaldehyde (OPA) method, copper method, immobilized enzyme biosensor method and/or others, comparing the results of the spectroscopic analysis with the results of the reference analysis, wherein preferably generating calibration data further includes one or several of the following steps: pre-processing the spectral signal for correcting and/or eliminating overlaying effects, wherein preferably pre-processing is conducted using multiplicative scatter correction (MSC), and/or first derivatives, and/or second derivatives, and/or smoothing, wherein preferably pre-processing is conducted before multiple and/or multivariate and/or linear regression analysis is carried out, and/or removing spectral signals that are not converted from electromagnetic waves that are reflected from or emitted through the sugar beets, preferably by differentiating the spectral signals using classification and/or filtering, in particular using mathematical filtering methods, and/or averaging spectral signals to one spectral signal, and/or carrying out multiple and/or multivariate and/or linear regression analysis for generating calibration data, wherein preferably the calibration data is derived using principle component analysis (PCA), and/or multiple linear regression (MLR), and/or partial least squares (PLS) regression, and/or machine learning, in particular using neuronal networks.

The sample of sugar beets can be a sample of sugar beet pieces. The sample of sugar beets can for example comprise 10 kg or 20 kg or 30 kg of sugar beets and/or sugar beet pieces. Other amounts of sugar beets and/or sugar beet pieces can also be used. Preferably, receiving electromagnetic waves can be understood as receiving electromagnetic waves that are reflected from the sample. Sugar beet pulp can be understood as a soft mass of mashed sugar beets. Reference analysis can comprise at least one or several measurements, wherein the at least one measurement can be conducted on the sugar beet pulp. Preferably, by comparing the results obtained from the spectroscopic analysis with the results obtained from the reference analysis, calibration data can be generated,

Preferably the spectroscopic analysis is carried out continuously. Preferably, calibration data is generated by repeatedly taking samples, conducting spectroscopic analyses of the samples and producing sugar beet pulp from the samples, Preferably, calibration data is generated by repeatedly comparing results of spectroscopic analyses with results from calibration analyses. It is preferred if this procedure is repeated several times, in particular more than 100 times, preferably more than 1000 times. Preferably, this procedure conducted on sugar beets with different components, preferably on relatively wet sugar beets, on relatively dry sugar beets, on sugar beets with high sugar content, on sugar beets with low sugar content, and/or on sugar beets with varying components.

Preferably, calibration data is generated by analysing sugar beet pieces on a bypass stream and/or by sampling of a defined amount of analysis portion in processes, in particular in a sugar production facility, preferably in regular time intervals. Preferably, calibration data is generated over a time period of at least several days, in particular of at least several weeks and/or of at least several months and/or of several years.

Generating calibration data can also be carried out in a static process, wherein the sample of sugar beets is not moved along a sensor. Preferably, within such a static process, the sample is located at a defined position, in particular under a sensor that is adapted to receive electromagnetic waves.

An advantage of generating calibration data in the described way is that the calibration data can be used for reliably determining components in sugar beets and/or sugar beet pieces, in particular even for sugar beets with different components and/or properties.

Preferably, generating calibration data comprises developing and/or optimising and/or validating a calibration model, wherein developing and/or optimising and/or validating the calibration model comprises generating a calibration set, a cross-validation set, and an independent validation set. Preferably, the calibration set comprises results obtained from spectroscopic analysis of a first part of the sample of sugar beets. Preferably, the cross-validation set comprises results obtained from spectroscopic analysis of a second part of the sample of sugar beets. Preferably, the independent validation set comprises results obtained from spectroscopic analysis of a third part of the sample of sugar beets. It is preferred if the first part, the second part, and the third part of the sample are different parts of the sample of sugar beets. Preferably, the first part, the second part and the third part are from different samples of sugar beets, in particular from samples of sugar beets that have been harvested at different locations. The calibration model can be generated from the calibration set. The cross-validation set can be used for developing, in particular improving, the calibration model. The independent validation set can be used for validating the calibration model. Outliers or also samples in validation set can be used to modify or to develop the calibration model. Preferably, the independent validation set is not used for changing and/or developing the calibration model.

According to a further preferred embodiment, the method comprises processing the spectral signal for determining components in at least the analysis portion and possibly the production portion, and/or comparing the spectral signal with the calibration data and dependent on the comparison determine, preferably quantitatively, the components in at least the analysis portion and possibly the production portion. Processing the spectral signal is carried out to determine components in at least the analysis portion, preferably in the analysis portion and at least a part of the production portion. The components in at least the analysis portion, and preferably in at least a part of the production portion, can for example be one or several of the following group: total sugar content, content of monosaccharides as glucose, fructose, galactose, content of disaccharides as sucrose, lactose, maltose, content of Oligosaccharides as raffinose, maltodextrin, cellodextrin, content of polysaccharides as inulins, fructans, extraction efficiency of sugar, dry matter content, crude protein, crude fiber, amino acids, starch, total sugar content, recoverable sugar content, soluble nitrogen compounds as proteins, betaine, betalaine, betacyanins, betaxanthins, amids and amino acids, insoluble nitrogen compounds as proteins, nitrogen free organic substances as pectins, saponins, organic acids, marc content, fat content, content of alcohols, content of structural carbohydrates as NDF (Neutral Detergent Fiber), ADF (Acid Detergent Fiber), ADL (Acid Detergent Lignin) or content of Hemicellulose, cellulose, Lignin, ash content, content of alkali metal elements and its inorganic compounds as sodium, sodium chloride, content of metal elements und inorganic compounds as calcium, calcium carbonate, magnesium, magnesium oxide, content of metalloid elements and inorganic compounds as boron, borat minerals, selenium, silicon, content of nonmetal elements and inorganic compounds as carbon, carbonates, phosphorus, phosphates, sulfur, iodine. The components can be determined qualitatively and/or quantitatively.

According to a further preferred embodiment of the method, the wavelength of the electromagnetic waves lies in the infrared spectrum, preferably in the near-infrared spectrum, and/or in the microwave region and/or in the visible spectrum, and/or in the ultraviolet spectrum, and/or the spectral signal is converted by using spectroscopy, in particular near-infrared spectroscopy (NIRS), mid- infrared-spectroscopy, far-infrared spectroscopy, terahertz-spectroscopy and/or ultraviolet-visible spectroscopy (UV-Vis) and/or Raman spectroscopy and/or laser-induced breakdown spectroscopy (LIBS), and/or fluorescence spectroscopy and/or hyperspectral imaging, and/or nuclear magnetic resonance and/or a combination of hyperspectral imaging with different spectroscopic approaches and/or combinations of different spectroscopic methods, wherein analysing is conducted using a camera and/or using a combination of a camera with different spectroscopic methods.

Preferably, the infrared spectrum comprises wavelengths in a range from 750 nm to 1,000,000 nm. Preferably, the near-infrared spectrum comprises wavelengths in a range from 750 nm to 2,500 nm. Preferably, the visible spectrum comprises wavelengths in a range from 400 nm to 750 nm. Further preferably, the wavelength of the electromagnetic waves lies in the ultraviolet spectrum, wherein preferably, the ultraviolet spectrum comprises wavelengths in a range from 10 nm to 400 nm.

Preferably, the spectral signal is converted by using spectroscopy, in particular near-infrared spectroscopy (NIRS), and/or mid infrared spectroscopy and/or far infrared spectroscopy and/or ultraviolet-visible spectroscopy (UV-Vis) and/or Raman spectroscopy and/or laser-induced breakdown spectroscopy (LIBS). In particular a combination of different spectroscopy methods as well in combination with hyperspectral images can be used, wherein preferably the spectral signals converted by using different spectroscopy methods can be combined for component analysis. Preferably, nuclear magnetic resonance may be also used in combination with any of the above-mentioned spectroscopical methods.

According to a further preferred embodiment, the method comprises changing at least one sugar production parameter, in particular electric field pulses and/or pulse numbers and/or process temperature and/or conveying speed and/or duration of the production portion in reactor dependent on the determined components in at least the analysis portion, and/or changing at least one drying process parameter for drying pressed pulp, in particular drying time and/or drying temperature for drying pressed pulp dependent on the determined components in at least the analysis portion, and/or changing at least the order in which sugar beets are introduced into a sugar production process dependent on the determined components in at least the analysis portion.

In beet juices purification milk of lime and CO2 can be used. Coke and limestone can be used for the production of CaO and CO2. The lime usage of the conventional process can be about 2% beet. Classical juice purification can consist of liming, carbonation, sludge separation and sulphitation. With this purification process parts of non-sugars can be removed from the sugar. The knowledge about quality of sugar beets can help to adjust the purifications process, in particular adjusting the amount of limestone added. An advantage of optimizing the addition of limestone lies in the fact that the costs can be reduced if a reduction of limestone is possible. Furthermore, analysis results of sugar beet quality loss during storage of sugar beets can be used to adjust the addition of limestone. In addition, the amount of water added during the extraction can be adjusted based on the quality determination of the beets. Relevant parameters can be content of sucrose and the content of non-sugar components as proteins, pectins, inorganic salts, organic acids, colouring substances as glucose and/or fructose, amino acid nitrogen and all soluble nitrogen compounds can negatively affect processing of sugar beet roots because of juice colour modifications and reduction of alkalinity.

Damaged sugar beets, which can be damaged defrosted sugar beets or otherwise damaged sugar beets can have a changed quality that may affect the filtration process during sugar extraction. Adding dextranase during sugar extraction can improve the filtration process. Analysing the presence and the amount of damaged sugar beets can be used for optimising the amount of dextranase addition/supplementation. With an optimization of dextranase addition the sugar beet extraction can be made more cost efficient.

In addition, analyzing the density of sugar beets can help to optimize the cutting and/or slicing process, for example by adjusting the sharpness of the cutting knives and/or by exchanging the cutting knives.

Sugar beets can be decapitated during harvesting, wherein the heads of the sugar beets can contain less sugar and more components that could negatively influence the sugar production process, such as for example nitrogen compounds, when compared to other parts of the sugar beets. In particular, the heads of the sugar beets can contain green material and leaves. Preferably, a determination of the amount of heads of sugar beets or the amount of green material in a sample of sugar beets can be used to estimate the quality of the sample of sugar beets and therefore to adjust the sugar production process dependent on the quality of the sample of sugar beets.

In sugar production, electric field pulses can be applied in an electrical reaction chamber, which is understood as a part of a device in which electric currents and/or electric fields act on target materials, in particular on sugar beet pieces, preferably at least the production portion of sugar beet pieces. An electrical reaction chamber is understood as an electroporation device, wherein electrical field pulses are applied to cells in order to increase the permeability of the cell membrane to improve the process of extracting sugar from at least the production portion. Pulse number refers to the number of electrical field pulses that are applied in the electrical reaction chamber within a certain period of time, in particular the number of electrical field pulses per second. Process temperature can be the temperature of a liquid, in particular water, within a chamber or reactor, preferably in an electrical reaction chamber, or the temperature of water introduced to a chamber, preferably an electrical reaction chamber. Conveying speed can be the speed of the stream of at least the analysis portion, wherein the stream of at least the production portion is generated by conveying at least the analysis portion along the transport direction using the transport device. The duration of the production portion in reactor is preferably the duration in which at least the production portion is arranged in a reactor, in particular in an electrical reaction chamber. A drying process parameter for drying pressed pulp can be the duration of drying pressed pulp and/or the temperature applied for drying pressed pulp.

The order in which sugar beets are introduced into a sugar production process can advantageously be regulated in such a way that the sugar production efficiency is increased. In particular, a waiting queue can be created dependent on the determined components in different sugar beets and/or different pluralities of sugar beets and/or different sugar beet pieces. Preferably, sugar beets are sorted dependent on analysed components of the sugar beets. Thus, advantageously sugar beets of similar quality can be sorted and processed together in a sugar production process in a sugar production facility, wherein the sugar production parameters can be adjusted to most efficiently extract sugar from the sugar beets.

According to a further preferred embodiment of the method, providing sugar beet pieces from the analysis portion is conducted using a chopping device, wherein the chopping device is configured to crumble and/or cut the analysis portion into substantially equal sized sugar beet pieces, and/or providing sugar beet pieces from the analysis portion and/or the production portion is conducted using a slicing device, wherein the slicing device is configured to cut the analysis portion and/or the production portion into sugar beet pieces that are formed as slices and/or thin elongated strips.

Chopping the analysis portion and/or production portion into sugar beet pieces can be conducted using a chopping device, wherein the chopping device is configured to cut and/or crumble the analysis portion and /or the production portion into substantially equal sized sugar beet pieces.

Preferably, the sugar beet pieces are chopped by crumpling sugar beets into sugar beet pieces, in particular using hooks that are adapted to crumble sugar beets into sugar beet pieces rather than cutting the sugar beets. By crumbling sugar beet pieces, sugar beet pieces are broken into pieces off the sugar beets, wherein the crumbled sugar beet pieces are rather dry and in particular do not comprise a flat and wet cutting surface.

Providing sugar beet pieces can also comprise slicing the analysis portion and/or the production portion into sugar beet pieces using a slicing device, wherein the slicing device is configured to cut and/or slice the analysis portion and/or the production portion into sugar beet pieces that are formed as slices and/or thin elongated strips.

Preferably, the production portion can be sliced and/or cut into sugar beet pieces by using a slicing device, wherein the slicing device is configured to cut the analysis portion and/or the production portion into sugar beet pieces that are formed as thin elongated strips. Preferably, the sugar beet pieces are cut, wherein the slicing device comprises blades and/or knifes for cutting and/or slicing sugar beets into sugar beet pieces. The sugar beet pieces formed as thin elongated strips can also be referred to as cossettes. Cutting sugar beets into sugar beet pieces that are formed as thin elongated strips is in particular advantageous because the surface area of the sugar beet pieces is relatively high, which can result in a good extractability of sugar from these sugar beet pieces that are formed as thin elongated strips. Preferably, the length of the sugar beet pieces that are formed as thin elongated strips is at least three times larger, in particular at least five times larger, than the thickness of the sugar beet pieces that are formed as thin elongated strips.

According to a further preferred embodiment of the method, the at least one transport device comprises a first transport section, a main transport section and a second transport section, wherein the main transport section is arranged downstream of the first transport section and upstream of the second transport section, and/or wherein the at least one transport device comprises a bypass section arranged downstream of the first transport section and upstream of the second transport section. Preferably the method further comprises conveying the production portion and the analysis portion along the first transport section, and/or conveying at least the analysis portion along the bypass section, and/or conveying at least a part of the production portion, in particular the sugar beet pieces that are not part of the analysis portion, along the main transport section, and/or conveying the production portion along the second transport section, and/or conveying the analysis portion along the second transport section and/or discarding the analysis portion.

Downstream and upstream are understood in a sense that when conveying at least the analysis portion using the at least one transport device along a transport direction a stream of at least the analysis portion is generated and the stream of at least the analysis portion moves along the transport direction from upstream to downstream. In particular, the stream of at least the analysis portion moves from upstream to downstream.

Preferably, the first transport section comprises an upstream end and a downstream end, the main transport section comprises an upstream end and a downstream end, and the second transport section comprises an upstream end and a downstream and, wherein the downstream end of the first transport section is connected to the upstream end of the main transport section and the downstream end of the main transport section is connected to the upstream end of the second transport section. The first transport section, the main transport section, and the second transport section can be sections of the same transport device. The first transport section, the main transport section, and the second transport section can be driven separately, in particular using electric motors.

It is further preferred that the transport device comprises a bypass section arranged downstream of the first transport section and upstream of the second transport section.

Preferably, the bypass section comprises an upstream end and a downstream end, wherein the upstream end is connected to the downstream end of the first transport section. Preferably, the analysis portion is conveyed along the bypass section from the upstream end of the bypass section to the downstream end of the bypass section. The downstream end of the bypass section can be connected to the upstream end of the second transport section.

According to a further preferred embodiment, the method comprises conveying the production portion and the analysis portion along the first transport section, and/or conveying at least the analysis portion along the bypass section, and/or conveying at least a part of the production portion, in particular the sugar beet pieces that are not part of the analysis portion, along the main transport section.

Preferably, the sugar beet pieces are divided, wherein the analysis portion is guided from the stream of sugar beet pieces on the first transport section to a bypass stream on the bypass section. Preferably, the bypass stream merges with the stream of the other sugar beet pieces at the second transport section. The bypass section can be arranged downstream of, and in particular adjacent to, the first transport section, and upstream of, and in particular adjacent to the second transport section. In particular, the bypass section connects the first transport section and the second transport section as an alternative transport route. Thus, a part of the sugar beet pieces, in particular the part that does not include the analysis portion, can be transported along the main transport section, and a part of the sugar beets, in particular the analysis portion, can be transported along the bypass transport section.

Preferably the transport velocity of the sugar beet pieces that are arranged on the bypass section is lower than, in particular less than half of, the transport velocity of the sugar beet pieces that are arranged on the main section and/or the first transport section and/or the second transport section.

Preferably, the method comprises conveying the production portion along the second transport section, and/or conveying the analysis portion along the second transport section and/or discarding the analysis portion.

Preferably, the production portion and the analysis portion are conveyed along the second transport section from the upstream end of the second transport section to the downstream end of the second transport section. In case that the analysis portion is not conveyed to the second transport section, the analysis portion can be discarded, in particular after conducting component analysis on the analysis portion on the bypass section.

According to a further preferred embodiment of the method, emitting electromagnetic waves towards at least the analysis portion is conducted while the analysis portion is arranged at, and preferably conveyed along, the first transport section and/or arranged at, and preferably conveyed along, the bypass section and/or wherein emitting electromagnetic waves towards the production portion is conducted while the production portion is arranged at, and preferably conveyed along, the first transport section.

Preferably, analysing components of at least the analysis portion is conducted on at least the analysis portion that is arranged on the bypass section. Analysing components of at least the analysis portion can alternatively or additionally be conducted on at least the analysis portion that is arranged on the first transport device or on the second transport device.

According to a further preferred embodiment, the method comprises homogeneously and/or heterogeneously distributing the sugar beet pieces onto the at least one transport device, preferably with a roller that is arranged above the at least one transport device or without a roller.

Preferably homogeneously distributing the sugar beet pieces onto the at least one transport device is conducted on at least the analysis portion when at least the analysis portion is arranged on the bypass section.

Homogeneously distributing the sugar beet pieces can be evenly distributing sugar beet pieces. Preferably, homogeneously distributing the sugar beet pieces is referred to distributing the sugar beet pieces in a way that the height of the sugar beet pieces on the at least one transport device is constant or varies in a range of preferably +/- 5 cm. The homogeneously distributed sugar beet pieces can be arranged on the at least one transport device, having a defined and constant height and/or width, wherein preferably variations can occur in the range of +/- 5 cm. In particular, the stream of sugar beet pieces that is generated from the transport device has a defined and constant height and/or width, wherein the direction of the width is referred to a direction orthogonal to the transport direction and parallel to a plane on which the sugar beet pieces are arranged on the at least one transport device, wherein the direction of the height is referred to a direction orthogonal to the transport direction and orthogonal to the plane on which the sugar beet pieces are arranged on the at least one transport device. The described process can be conducted in a similar way for heterogeneously distributing of sugar beet pieces as for example for directly measurements on cossettes or dried pulp. Preferably, no defined and/or constant hight or width is needed. Preferably, homogenously distributing sugar beets by using a roller and/or heterogeneously distributing sugar beets without a roller can be conducted.

Preferably, homogeneously distributing is conducted by using a roller, wherein preferably the roller is arranged with a roller axis at a fixed and constant distance above the at least one transport device. Preferably, at least the analysis portion is compressed using the roller to a certain height, wherein in particular the surface of at least the analysis portion is smooth and even. The roller can be driven to rotate by the stream of at least the analysis portion and/or by a motor, wherein the roller can be driven with or in opposite direction to the stream of at least the analysis portion that moves along the transport direction.

According to a further preferred embodiment of the method, the components in the analysis portion preferably comprise one or several of the following group, in particular when analysing at least the analysis portion and possibly the production portion is conducted after producing pressed pulp: dry matter content, protein content, carbohydrate content, fiber content, content of cellulose; content of hemicellulose; neutral detergent fiber (NDF) content, acid detergent fiber (ADF) content, ADL (Acid Detergent Lignin) content; lignin content, amino acid content, starch content, total sugar content, content of monosaccharides; content of oligosaccharides; content of polysaccharides; gross energy content, feed unit for milk production (UFL), metabolizable energy (ME), ash content. Preferably, the components in the analysis portion preferably comprise one or several of the following group, in particular when analysing at least the analysis portion and possibly the production portion is conducted after providing sugar beet pieces, in particular by slicing, and preferably prior to producing a raw juice from at least the production portion: total sugar content, content of monosaccharides as glucose; fructose; galactose; content of disaccharides as sucrose, lactose, maltose; content of Oligosaccharides as raffinose, maltodextrin, cellodextrin; content of polysaccharides as inulins, fructans; extraction efficiency of sugar; dry matter content, crude protein, crude fiber, amino acids, starch, total sugar content, recoverable sugar content, soluble nitrogen compounds as proteins, betaine, betalaine, betacyanins, betaxanthins, amids and amino acids; insoluble nitrogen compounds as proteins; nitrogen free organic substances as pectins, saponins, organic acids; marc content; fat content; content of alcohols; content of structural carbohydrates as NDF (Neutral Detergent Fiber); ADF (Acid Detergent Fiber); ADL (Acid Detergent Lignin) or content of Hemicellulose; cellulose; Lignin; ash content; content of alkali metal elements and its inorganic compounds as sodium; sodium chloride; content of metal elements und inorganic compounds as calcium; calcium carbonate, magnesium, magnesium oxide; content of metalloid elements and inorganic compounds as boron, borat minerals, selenium, silicon; content of nonmetal elements and inorganic compounds as carbon, carbonates, phosphorus, phosphates; sulfur, iodine.

According to a further preferred embodiment, the method comprises changing at least one sugar production parameter, in particular electric field pulses and/or pulse numbers and/or process temperature and/or conveying speed and/or duration of the production portion in reactor dependent on the determined components in the pressed pulp analysis portion, and/or changing at least one drying process parameter for drying pressed pulp, in particular drying time and/or drying temperature for drying the pressed pulp dependent on the determined components in the pressed pulp analysis portion, and/or changing at least the order in which sugar beets are introduced into a sugar production process dependent on the determined components in the pressed pulp analysis portion. Regarding the sugar production parameters, it is referred to the definitions and preferred embodiments as described above.

According to a further aspect, it is provided a method for generating calibration data for the determination of components in sugar beets for sugar production, comprising taking a sample of sugar beets, preferably a determined amount, for example 10kg, and preferably moving the sample along a sensor that is adapted to receive electromagnetic waves, spectroscopic analysis of the sample by, preferably continuously, emitting electromagnetic waves towards the sample, receiving electromagnetic waves, and converting the received electromagnetic waves into a spectral signal, producing a sugar beet pulp from the sample, preferably by a cutter mill, and extracting the sugar beet pulp, preferably by Aluminium sulfate or lead acetate solution or water, reference analysis of the extracted sugar beet pulp by conducting measurements, such as polarimetry, flame photometry, fluorometric o-phthalaldehyde (OPA) method, copper method, immobilized enzyme biosensor method and/or others, comparing the results of the spectroscopic analysis with the results of the reference analysis.

Further advantageous embodiments of the method described above can be realized by combining some or all of the preferred features described herein.

According to a further aspect, it is provided an arrangement for determining components in sugar beets for sugar production, comprising a receiving section for receiving a plurality of sugar beets including a production portion of sugar beets for sugar production and an analysis portion of sugar beets for component analysis and possibly for sugar production, a chopping device, wherein the chopping device is configured to crumble and/or cut the analysis portion into substantially equal sized sugar beet pieces and/or a slicing device, wherein the slicing device is configured to cut the analysis portion and/or the production portion into sugar beet pieces that are formed as slices and/or thin elongated strips, a transport device for conveying at least the analysis portion and possibly the production portion, an analysis assembly arranged to emit electromagnetic waves towards at least the analysis portion that is arranged on the transport device, wherein preferably the analysis assembly is arranged to receive electromagnetic waves, and/or wherein preferably the analysis assembly is arranged to convert the received electromagnetic waves into a spectral signal, a raw juice production device for producing raw juice from at least the production portion, and/or a control unit for controlling the analysis assembly and/or for receiving data from the analysis assembly, wherein preferably the control unit is arranged for changing at least one sugar production parameter, in particular electric field pulses and/or pulse numbers and/or process temperature and/or conveying speed and/or duration of the production portion in reactor, and/or application of milk of lime and CO2 in raw juice purification and or adjustment of processes of liming, carbonation, sludge separation and sulphitation in juice purification dependent on components determined by the analysis assembly, and/or changing at least one drying process parameter for drying pressed pulp, in particular drying time and/or drying temperature for drying pressed pulp dependent on components determined by the analysis assembly, and/or changing at least the order in which sugar beets are introduced into a sugar production process dependent on components determined by the analysis assembly.

In a sugar production process, ammonium can be generated from nitrogen compounds, wherein the ammonium can be volatile and can decrease air quality inside the sugar production facility and outside of the sugar production facility. Preferably, with an analysis of the quality of the sugar beets, the sugar production parameters can be adjusted in a way that the ammonium content in the air and can be decreased and air quality can be increased, which can be beneficial for persons inside the sugar production factory and for the environmental impact of the sugar production facility. Preferably, by adjusting the sugar production parameters, CO2 emissions can be reduced. During the extraction of sugar, for example adding water and/or changing the cutting process of the sugar beets and/or changing the time of the sugar extraction can be adjusted.

The arrangement preferably comprises a mechanical press, in particular a pulp press that is specifically designed for pressing sugar beet pieces, for extracting liquid from the sugar beet pieces. The receiving section can be a section and/or station for receiving sugar beets from transport vehicles, such as for example trucks and/or trains. The receiving section can be an inlet of the chopping device, wherein the sugar beets are introduced to the chopping device through the inlet. The receiving section can be an inlet of the slicing device, wherein the sugar beets are introduced to the slicing device through the inlet. Preferably, the analysis assembly comprises an electromagnetic wave source for emitting electromagnetic waves towards at least the analysis portion that is arranged on the at least one transport device. Preferably, the analysis assembly comprises a sensor for receiving reflected electromagnetic waves. Preferably, the analysis assembly comprises a spectrometer, in particular a spectrometer that is adapted to carry out near-infrared spectroscopy (NIRS); and/or mid- infrared-spectroscopy; and/or far-infrared- spectroscopy; and/or ultraviolet-visible spectroscopy (UV-Vis) and/or Raman spectroscopy and/or laser-induced breakdown spectroscopy (LIBS) and/or Hyperspectral Imaging; and/or in combination. The electromagnetic wave source can be part of the spectrometer or not part of the spectrometer. The sensor can be part of the spectrometer or not part of the spectrometer. Nuclear magnetic resonance can be also used in combination with any of the above-mentioned spectroscopical methods.

According to a preferred embodiment, the arrangement comprises a control unit for controlling the analysis assembly and/or for receiving data from the analysis assembly, wherein preferably the control unit is arranged for changing at least one sugar production parameter, in particular electric field pulses and/or pulse numbers and/or process temperature and/or conveying speed and/or duration of the production portion in reactor dependent on components determined by the analysis assembly, and/or changing at least one drying process parameter for drying pressed pulp, in particular drying time and/or drying temperature for drying pressed pulp dependent on components determined by the analysis assembly, and/or changing at least the order in which sugar beets are introduced into a sugar production process dependent on components determined by the analysis assembly.

The control unit can receive data and/or signals from the analysis assembly. The control unit is adapted to change at least one, preferably several, sugar production parameters dependent on the received data and/or on the received signals. Thus, the sugar production parameters can be adjusted by changing sugar production parameters that are tailored to the sugar beet pieces that are currently in the sugar production facility. An advantage of changing sugar production parameters in such a way is that sugar can be extracted more efficiently from the sugar beet pieces.

According to a further aspect, it is provided a sugar production facility, comprising an arrangement as described herein.

According to a further aspect, it is provided the use of an analysis assembly in a sugar production facility, in particular a sugar production facility as described herein, and/or the use of an arrangement for determining components in sugar beets for sugar production, in particular an arrangement for determining components in sugar beets for sugar production as described herein, in a sugar production facility, in particular a sugar production facility according to the preceding claim, and/or the use of a method for determining components in sugar beets for sugar production, in particular a method for determining components in sugar beets for sugar production as described herein, in a sugar production facility, in particular a sugar production facility as described herein.

The further aspects described above and their respective possible embodiments comprise features and/or method steps that are particularly suitable to be used with and/or connected with the method and its preferred embodiments described herein.

For the advantages, preferred embodiments, and details of the individual different aspects and their preferred embodiments, reference is also made to the description, and in particular to the described advantages, preferred embodiments, and details described with reference to the respective other aspects.

Further advantageous embodiments result from the combination of individual, several or all of the preferred features described herein.

Preferred embodiments shall now be described with reference to the attached drawings, in which
- Fig. 1: shows a schematic representation of an example of a method for determining components in sugar beets for sugar production;
- Fig. 2: shows a preferred example of a sugar production facility with an arrangement for determining components in sugar beets for sugar production;
- Fig. 3: shows an example of a spectral signal obtained from using near-infrared spectroscopy on sugar beet pieces for sugar production;
- Fig. 4a: shows a first example of a transport device in a sugar production facility with a bypass section;
- Fig. 4b: shows a second example of a transport device in a sugar production facility with a bypass section;
- Fig. 4c: shows a third example of a transport device in a sugar production facility with a bypass section;
- Fig. 5: shows a diagram showing polarisation measured with near-infrared spectroscopy versus polarisation measured with reference method;
- Fig. 6: shows a combination of different spectroscopy methods for component analysis;
- Fig. 7: shows 11 segments of a sugar beet for investigating heterogeneity in sugar beets;
- Fig. 8: shows a schematic representation of an example of a method for generating calibration data for the determination of components in sugar beets for sugar production.

In the figures, elements with the same or comparable functions are indicated with the same reference signs.

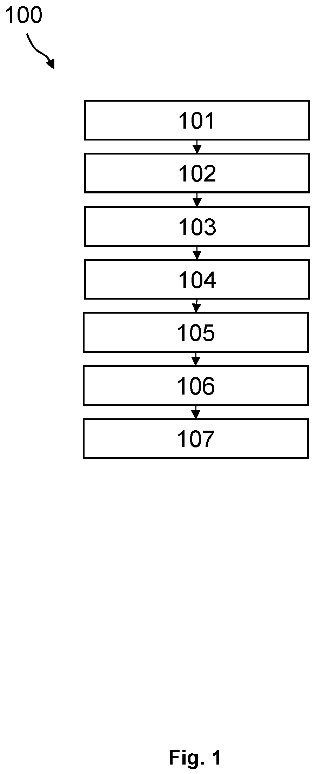

Fig. 1 shows a schematic representation of an example of a method 100 for determining components in sugar beets for sugar production. The method 100 comprises the steps described in the following. In a step 101, receiving a plurality of sugar beets including a production portion of sugar beets for sugar production and an analysis portion of sugar beets for component analysis and possibly for sugar production. In a step 102, providing the plurality of sugar beets including a production portion of sugar beets for sugar production and an analysis portion of sugar beets for component analysis and possibly for sugar production. Preferably, the production portion and/or the analysis portion are provided in sugar beet pieces by chopping and/or slicing. In a step 103, analysing at least the analysis portion and possibly the production portion. In a step 104, emitting electromagnetic waves towards at least the analysis portion and possibly the production portion. In a step 105, receiving electromagnetic waves. In a step 106, converting the received electromagnetic waves into a spectral signal. In a step 107, producing sugar from at least the production portion and possibly from the analysis portion.

An example of the method for determining components in sugar beets for sugar production can be as follows, wherein the order of the steps can be different from the order in which the steps are described in the following. The method can comprise the steps described in Fig. 1 and one or several of the steps described in following, wherein the order of the steps can be different from the order in which the steps are described in the following. In a step, providing sugar beet pieces and/or slices from the production portion and/or the analysis portion. In a step, arranging at least the analysis portion and possibly the production portion onto at least one transport device. In a step, conveying at least the analysis portion and possibly the production portion using the at least one transport device, preferably with a transport velocity within a range of 0.05 m/s to 20 m/s, in particular 0.05 m/s to 10 m/s, preferably within a range of 0.05 m/s to 5 m/s, more preferably within a range of 0.05 m/s to 1 m/s. In a step, producing pressed pulp by removing liquid substances from at least the production portion and/or at least the analysis portion, preferably using a mechanical press. In a step, generating calibration data, including taking a sample of sugar beets, preferably a determined amount, for example 10 kg, spectroscopic analysis of the sample by, preferably continuously, emitting electromagnetic waves towards the sample, receiving electromagnetic waves, and converting the received electromagnetic waves into a spectral signal, producing a sugar beet pulp from the sample, preferably by a cutter mill, and extracting the sugar beet pulp, preferably by Aluminium sulfate or lead acetate solution or water, reference analysis of the extracted sugar beet pulp by conducting measurements, such as polarimetry, flame photometry, fluorometric o-phthalaldehyde (OPA) method, copper method, immobilized enzyme biosensor method and/or others, comparing the results of the spectroscopic analysis with the results of the reference analysis. In a step, processing the spectral signal for determining components in at least the analysis portion and possibly the production portion. In a step, comparing the spectral signal with the calibration data and dependent on the comparison determine, preferably quantitatively, the components in at least the analysis portion and possibly the production portion. In a step, changing at least one sugar production parameter, in particular electric field pulses and/or pulse numbers and/or process temperature and/or conveying speed and/or duration of the production portion in reactor dependent on the determined components in at least the analysis portion. In a step, changing at least one drying process parameter for drying pressed pulp, in particular drying time and/or drying temperature for drying pressed pulp dependent on the determined components in at least the analysis portion. In a step, changing at least the order in which sugar beets are introduced into a sugar production process dependent on the determined components in at least the analysis portion. In a step, conveying the production portion and the analysis portion along the first transport section. In a step, conveying at least the analysis portion along the bypass section, wherein the bypass section can be used for analysing the analysis portion and/or for analysing the production portion and/or as step in a calibration process. In a step, conveying at least a part of the production portion, in particular the sugar beet pieces that are not part of the analysis portion, along the main transport section. In a step, conveying the production portion along the second transport section. In a step, conveying the analysis portion along the second transport section and/or discarding the analysis portion. In a step, homogeneously distributing the sugar beet pieces onto the at least one transport device, preferably with a roller that is arranged above the transport device or heterogeneously distributing the sugar beet pieces onto at least one transport device without using a roller.

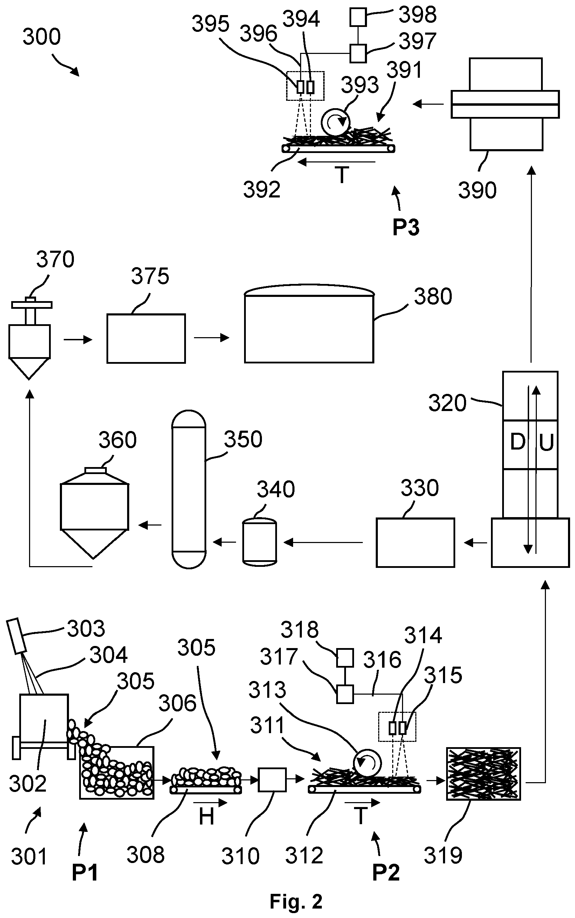

Fig. 2 shows a preferred example of a sugar production facility 300 with an arrangement for determining components in sugar beets for sugar production. Harvested sugar beets are received at a receiving section 301 of the sugar production facility. The sugar beets are transported to the receiving section 301 by a truck 302. At the receiving section 301, the sugar beets 305 are removed from the truck 302 with a water jet 304 that is generated from a water source 303. At the receiving section 301, the sugar beets can be analysed at a first analysis position P1. At the first analysis position P1 the sugar beets can be analysed with the method described herein. Then, the sugar beets 305 are washed using a sugar beet washer 306 to remove soil, stones, mud and sand. The beet washer 306 can also contain a magnet to remove metal parts. Subsequently, the sugar beets 305 are conveyed along a hopping direction H to a slicing device 310 using a sugar beet hopper 308. With the slicing device 310 the sugar beets 305 are cut into sugar beet pieces, wherein the slicing device cuts the sugar beets 305 into sugar beet pieces 311 that are formed as thin elongated strips. The sugar beet pieces 311 are arranged on a transport device 312, which can be a conveyor belt. The sugar beet pieces 311 are transported in a transport direction T from an upstream end of the transport device to a downstream end of the transport device, wherein the transport device 312 generates a stream of sugar beet pieces that moves along the transport direction T. A roller 313 can be arranged above the transport device that is adapted to homogeneously distribute and compact the sugar beet pieces that move on the transport device 312 along the transport direction T. However, it is also possible to use the method described herein without a roller.

At a second analysis position P2 sugar beet pieces can be analysed with the method described herein. Above the transport device 312, an analysis assembly is arranged. Wherein the analysis assembly is arranged to emit electromagnetic waves towards the sugar beet pieces 311 that are arranged on the transport device 312 by using an electromagnetic wave source 314. Furthermore, the analysis assembly is arranged to receive reflected electromagnetic waves by using an optical system 315 that is located directly downstream of the roller 313. The optical system 315 is arranged for detecting the reflected electromagnetic waves from the surface of the sugar beet pieces 312. Furthermore, the analysis assembly is arranged to convert the received reflected electromagnetic waves into a spectral signal. The optical system 315 can continuously record reflected electromagnetic waves and transmit them via an optical fiber 316 to a spectrometer 317, which converts the spectrally resolved reflected electromagnetic wavelengths into spectral signals, for example in the wavelength range from 850 nm to 1650 nm. During the stream of sugar beet pieces several of such spectral signals are produced, which can be filtered and averaged by a processor 318. By comparison with suitable calibration data, the components of the sugar beet pieces such as for example extractable sugar content and several others can be accurately determined. The processer 318 can comprise a control unit for controlling the analysis assembly and for receiving data from the analysis assembly. The control unit is arranged for changing at least one sugar production parameter dependent on components determined by the analysis assembly. Thus, the sugar production process can be directly adapted to the components of the sugar beet pieces that are currently in the sugar production facility in the sugar production process. Hence, with such an in-process analysis of components, the sugar production process can be optimally adjusted according to the sugar beet pieces from which sugar shall be extracted.

The sugar beet pieces 311 are then transported to a sugar beet pieces mixer 319 where the sugar beet pieces are mixed with hot juice. Subsequently, the sugar beet pieces are pumped into the bottom of a diffuser 320. The sugar is extracted from the sugar beet pieces by means of hot water (for example with a water temperature of about 70 °C) in the diffuser. In the diffuser 320, the sugar beet pieces move in an upwards direction U and the hot water moves in a downwards direction D, wherein the hot water moves in opposite direction of the direction of the movement of the sugar beet pieces. In the diffuser, sugar is extracted from the sugar beet pieces and obtained from the hot water. This results in a raw juice. The raw juice can contain about 98 wt.% of the total sugar content of the sugar beets and organic and inorganic constituents, which are called non-sugars, from the sugar beets. The non-sugars in the raw juice are bound and extracted by means of the natural substances lime and carbonic acid gas at a lime kiln in a juice cleaning arrangement 330. Subsequently, the flocculatable insoluble non-sugars and the lime are filtered out in filter units 340. The filtrate is referred to as thin juice and the filter residue as carbonated lime. The thin juice is thickened in a course of a multi-stage evaporation process with evaporators 350. This results in a thick juice. The thick juice is thickened further in the evaporators under vacuum. Subsequently, a crystallization process is triggered by adding finely ground sugar to the thick juice in a crystallization device 360. This results in a syrup. Then, sugar crystals are separated from the syrup by means of centrifugation using a centrifuge 370. This results in sugar. The sugar is dried by using a drying device 375 that applies an air stream on the sugar. Subsequently, the dried sugar can be stored in a silo 380.

From the diffuser 320 the sugar beet pieces from which sugar has been extracted in the diffuser 320 are transported to a mechanical press 390. The mechanical press 390 presses the sugar beet pieces from which sugar has been extracted to remove liquid from the sugar beet pieces. This results in pressed pulp. The pressed pulp has a lower moisture content compared to the sugar beet pieces before pressing the sugar beet pieces. At a third analysis position P3 the pressed pulp can be analysed with the method described herein. From the mechanical press 390 the pressed pulp is transported to another analysis assembly, wherein this analysis assembly is arranged to emit electromagnetic waves towards the pressed pulp 391 that are arranged on another transport device 392 by using an electromagnetic wave source 394. Furthermore, the analysis assembly is arranged to receive reflected electromagnetic waves by using an optical system 395 that is located directly downstream of a roller 393, wherein the method can also be applied without a roller. The optical system 395 is arranged for detecting the reflected electromagnetic waves from the surface of the pressed pulp 391. Furthermore, the analysis assembly is arranged to convert the received reflected electromagnetic waves into a spectral signal. The optical system 395 can continuously record reflected electromagnetic waves and transmit it via an optical fiber 396 to a spectrometer 397, which converts the spectrally resolved reflected electromagnetic wavelengths into spectral signals, for example in the wavelength range from 850 nm to 1650 nm. During the stream of the pressed pulp 391 a plurality of such spectral signals is produced, which can be filtered and averaged by a processor 398. By comparison with suitable calibration data, the components of the pressed pulp, such as for example remaining moisture content and several others, can be accurately determined. The processor 398 can comprise a control unit for controlling the analysis assembly and for receiving data from the analysis assembly. The control unit is arranged for changing at least one sugar production parameter and/or at least one parameter of the mechanical press dependent on components of the pressed pulp determined by the analysis assembly.

In this example, at the analysis positions P2, all sugar beet pieces are analysed for component analysis and all sugar beet pieces are used for sugar production. Therefore, in this example, all sugar beet pieces are part of the analysis portion and all sugar beet pieces are part of the production portion. Furthermore, in this example, the whole pressed pulp that is produced is analysed for pressed pulp component analysis. Therefore, in this example, all of the pressed pulp is part of the pressed pulp analysis portion.

In addition to the analysis positions P1, P2, and P3, sugar beets can also be analysed with the method described herein at other analysis position, such as a fourth analysis position that is arranged at a storage section where sugar beets are stored.

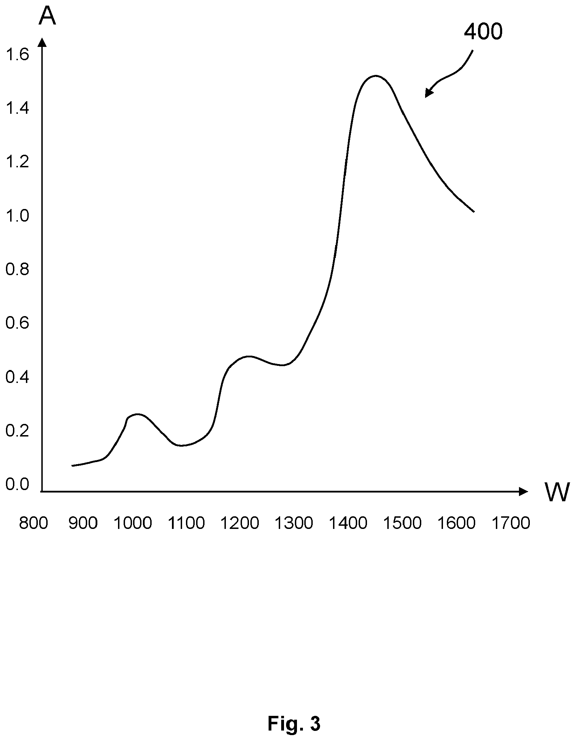

Fig. 3 shows an example of a spectral signal 400 obtained from using near-infrared spectroscopy on sugar beet pieces for sugar production. To generate a spectral signal 400, first, electromagnetic waves are emitted towards sugar beet pieces that are arranged on the transport device by using an electromagnetic wave source. Then, reflected electromagnetic waves are received by using a sensor, wherein the sensor is arranged for detecting the reflected electromagnetic waves from the surface of the sugar beet pieces. Then, the received reflected electromagnetic waves are converted into a spectral signal using a near-infrared spectrometer. In the shown example, reflected electromagnetic waves in the wavelength range from 850 nm to 1650 nm are received. In the shown diagram, the absorbance A is plotted versus the wavelength W (in nm). The spectral signal 400 represents atypical curve representing a spectral signal of sugar beet pieces. During a stream of sugar beet pieces several of such spectral signals can be produced, which can be filtered and averaged by a processor. From such a spectral signal 400 or several of such spectral signals, it is possible to determine components in sugar beet pieces.

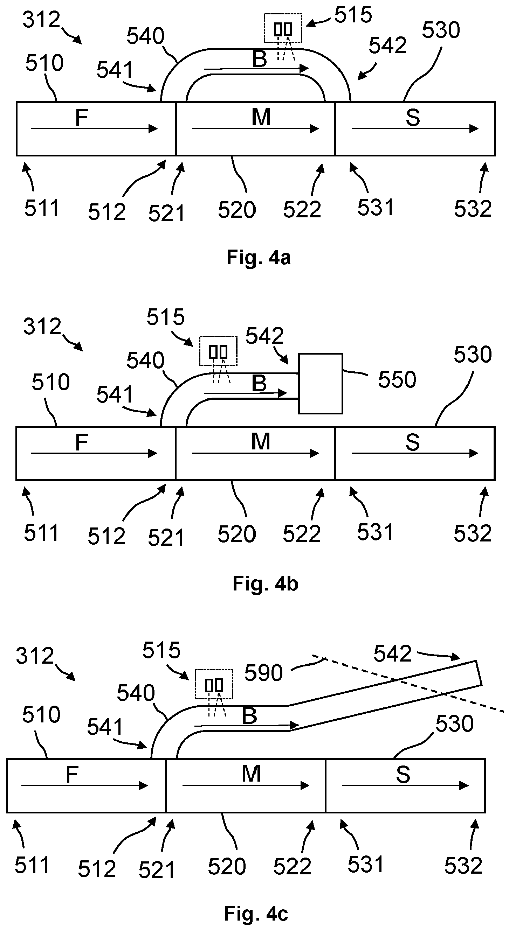

Fig. 4a shows a first example of a transport device 312 in a sugar production facility with a bypass section 540. The transport device 312 comprises a first transport section 510 that is arranged upstream of the transport device 312 and that is arranged to convey sugar beet pieces, comprising an analysis portion for component analysis and a production portion for sugar production, along a transport direction F from an upstream end 511 of the first transport section 510 to a downstream end 512 of the first transport section 510.

Adjacent to the first transport section 510, a main transport section 520 is arranged, wherein the main transport section 520 has an upstream end 521 and a downstream end 522 and the upstream end 521 of the main transport section 520 is arranged adjacent to the downstream end 512 of the first transport section 510. Optionally a roller can be arranged at first transport section. The main transport section 520 conveys a part of the production portion, except for the part of the production portion that is the analysis portion, along a transport direction M from the upstream end 521 of the main transport section 520 to the downstream end 522 of the main transport section 520.

The bypass section 540 has an upstream end 541 and a downstream end 542, wherein the analysis portion is conveyed in a transport direction B from the upstream end 541 of the bypass section 540 to the downstream end 542 of the bypass section 540. Above the bypass section 540, an analysis assembly 515 is arranged, wherein the analysis assembly 515 is arranged to emit electromagnetic waves towards the analysis portion that is arranged on the bypass section 540, wherein the analysis assembly 515 is arranged to receive reflected electromagnetic waves, and wherein the analysis assembly 515 is arranged to convert the received reflected electromagnetic waves into a spectral signal. Optionally a roller can be arranged at the bypass section.

Adjacent to the main transport section 520 and the bypass section 540, a second transport section 530 is arranged. The second transport section 530 has an upstream end 531 and a downstream end 532. Sugar beet pieces including the analysis portion and the production portion are conveyed along a transport direction S from an upstream end 531 of the second transport section 530 to a downstream end 532 of the second transport section 530.

As described in this example, the analysis portion is divided from a main stream of sugar beet pieces, which is a stream of the production portion and the analysis portion, via the bypass section 540 at the downstream end 512 of the first transport section 510. Then, the analysis portion is analysed on the bypass section 540 using the analysis assembly 515. Subsequently, the analysis portion is reintroduced to the main stream at the upstream end 531 of the second transport section 530. Thus, in this example, the analysis portion is part of the production portion.

Fig. 4b shows a second example of a transport device 312 in a sugar production facility with a bypass section 540. The transport device 312 comprises a first transport section 510 that is arranged upstream of the transport device 312 and that is arranged to convey sugar beet pieces, comprising an analysis portion for component analysis and a production portion for sugar production, along a transport direction F from an upstream end 511 of the first transport section 510 to a downstream end 512 of the first transport section 510.

Adjacent to the first transport section 510, a main transport section 520 is arranged, wherein the main transport section 520 has an upstream end 521 and a downstream end 522 and the upstream end 521 of the main transport section 520 is arranged adjacent to the downstream end 512 of the first transport section 510. The main transport section 520 conveys the production portion along a transport direction M from the upstream end 521 of the main transport section 520 to the downstream end 522 of the main transport section 520.

The bypass section 540 has an upstream end 541 and a downstream end 542, wherein the analysis portion is conveyed in a transport direction B from the upstream end 541 of the bypass section 540 to the downstream end 542 of the bypass section 540. Above the bypass section 540, an analysis assembly 515 is arranged, wherein the analysis assembly 515 is arranged to emit electromagnetic waves towards the analysis portion that is arranged on the bypass section 540, wherein the analysis assembly 515 is arranged to receive reflected electromagnetic waves, and wherein the analysis assembly 515 is arranged to convert the received reflected electromagnetic waves into a spectral signal. Adjacent to the downstream end 542 of the bypass section 540, a container 550 for collecting the analysis portion is arranged, wherein the analysis portion is conveyed into the container 550. The analysis portion in the container 550 can for example be discarded or be reintroduced into the sugar production process or be used in a different way. Optionally a roller can be arranged at the bypass section.

Adjacent to the main transport section 520, a second transport section 530 is arranged. The second transport section 530 has an upstream end 531 and a downstream end 532. The production portion is conveyed along a transport direction S from an upstream end 531 of the second transport section 530 to a downstream end 532 of the second transport section 530.

As described in this example, the analysis portion is divided from the main stream via the bypass section 540 at the downstream end 512 of the first transport section 510, analysed on the bypass section 540 using the analysis assembly 515, and then introduced into the container 550. Thus, in this example, the analysis portion is not part of the production portion.

Fig. 4c shows a third example of a transport device 312 in a sugar production facility with a bypass section 540. The transport device 312 comprises a first transport section 510 that is arranged upstream of the transport device 312 and that is arranged to convey sugar beet pieces, comprising an analysis portion for component analysis and a production portion for sugar production, along a transport direction F from an upstream end 511 of the first transport section 510 to a downstream end 512 of the first transport section 510.

Adjacent to the first transport section 510, a main transport section 520 is arranged, wherein the main transport section 520 has an upstream end 521 and a downstream end 522 and the upstream end 521 of the main transport section 520 is arranged adjacent to the downstream end 512 of the first transport section 510. The main transport section 520 conveys the production portion along a transport direction M from the upstream end 521 of the main transport section 520 to the downstream end 522 of the main transport section 520.

The bypass section 540 has an upstream end 541 and a downstream end 542, wherein the analysis portion is conveyed in a transport direction B from the upstream end 541 of the bypass section 540 to the downstream end 542 of the bypass section 540. Above the bypass section 540, an analysis assembly 515 is arranged, wherein the analysis assembly 515 is arranged to emit electromagnetic waves towards the analysis portion that is arranged on the bypass section 540, wherein the analysis assembly 515 is arranged to receive reflected electromagnetic waves, and wherein the analysis assembly 515 is arranged to convert the received reflected electromagnetic waves into a spectral signal. The downstream end 542 of the bypass section 540 is arranged outside of the sugar production facility. The line 590 indicates a border of the sugar production facility. Optionally a roller can be arranged at the bypass section.

Adjacent to the main transport section 520, a second transport section 530 is arranged. The second transport section 530 has an upstream end 531 and a downstream end 532. The production portion is conveyed along a transport direction S from the upstream end 531 of the second transport section 530 to the downstream end 532 of the second transport section 530.

As described in this example, the analysis portion is divided from the main stream via the bypass section 540 at the downstream end 512 of the first transport section 510, analysed on the bypass section 540 using the analysis assembly 515, and then conveyed outside of the sugar production facility. Thus, in this example, the analysis portion is not part of the production portion.

A transport device 392 for conveying pressed pulp can also comprise a bypass section as described in one of the examples that are shown in Fig. 4a, 4b and 4c and be designed accordingly.

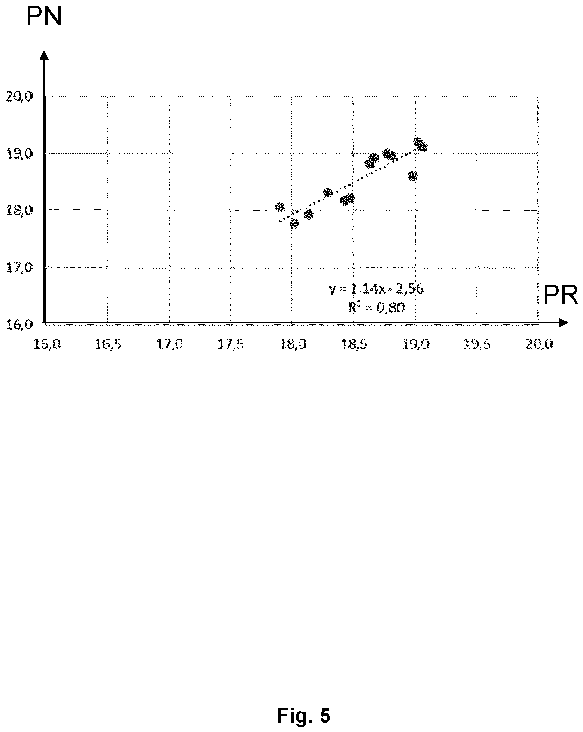

Fig. 5 shows a diagram in which polarisation measured on sugar beet pieces using near-infrared spectroscopy PN is plotted versus polarisation measured with a reference method PR. A calibration was developed for sugar beet pieces to check the performance in component analysis on sugar beet pieces that are formed as slices, i.e. as thin elongated strips. Samples consisting of 10 kg of sugar beet pieces in form of slices were analysed using NIRS analysis. Therefore, 10 kg of sugar beet pieces from a sugar production process were taken, then a stream of slices with a height of 100 mm and speed of 0.1 m/s on a conveyor belt was generated. Then the slices were continuously irradiated with electromagnetic waves. Then, reflected radiation in a wavelength range of 850 - 1650nm was continuously recorded every 45 ms. Then, the recorded radiation was converted into a spectral signal, and the spectral signal was processed for determination of polarisation in the sugar beet pieces. After such an analysis using NIRS, the sample consisting of 10 kg slices was cut in a cutting mill and mill pulp was produced from the sample. This mill pulp was homogenized, and a subsample was taken and frozen at -25 °C. The frozen pulp samples were extracted with warm water (with a temperature of about 40 °C) and the polarisation was determined using a polarimeter. The predicted results from the NIRS analysis PN were compared with values of polarization obtained by using the polarimeter PR. It can be seen that the values for the polarisation obtained using NIRS can be matched with the values for polarisation obtained using a polarimeter. When using a linear equation, as shown in the diagram, the correlation is R²=0.8. By comparing the values for the polarisation obtained from these two different analysis methods, calibration data as described herein can be generated. These results show that component analysis using spectroscopy directly on sugar beet pieces that are present in a sugar production facility is possible.

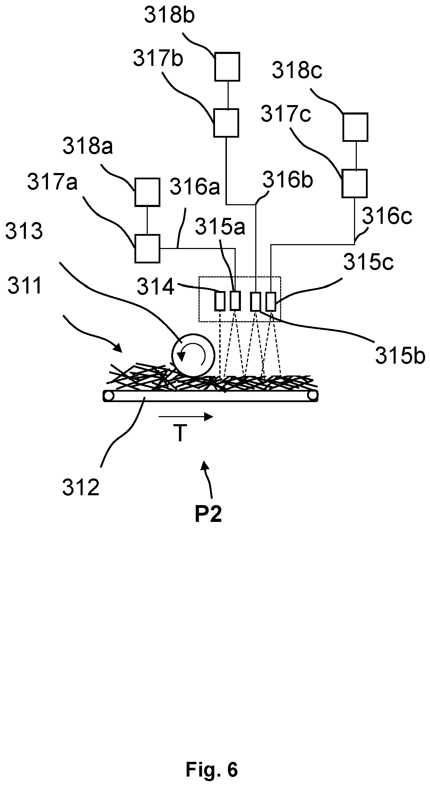

Fig. 6 shows an example for a combination of different spectroscopy methods for component analysis. Sugar beet pieces at the second analysis position P2 as described in Fig. 2 are shown. The difference in respect to Fig. 2 lies in a combination of different spectroscopy methods. Here, three optical systems 315a, 315b, 315c are arranged for detecting reflected electromagnetic waves from the surface of the sugar beet pieces 312. One optical system 315a is arranged for detecting electromagnetic waves in a wavelength range that lies in the near-infrared spectrum. Another optical system 315b is arranged for detecting electromagnetic waves in a wavelength range that lies in a spectrum suitable for Raman spectroscopy. Another optical system 315c is arranged for detecting electromagnetic waves in a wavelength range that lies in a spectrum suitable for LIBS spectroscopy. For the optical systems 315b and 315c individual light sources can be used. The received reflected electromagnetic waves are converted into spectral signals. The optical systems 315a, 315b, 315c can continuously record reflected electromagnetic waves and transmit the data via optical fibers 316a, 316b, 316c to spectrometers 317a, 317b, 317c, which convert the spectrally resolved reflected electromagnetic wavelengths into spectral signals. One of the spectrometers 317a is a NIR spectrometer, another spectrometer 317b is a LIB spectrometer, and another spectrometer 317c is a Raman spectrometer. During the stream of sugar beet pieces several of such spectral signals are produced, which can be filtered and averaged by processors 318a, 318b, 318c. By comparison with suitable calibration data, the components of the sugar beet pieces such as for example extractable sugar content and several others can be accurately determined. The processers 318a, 318b, 318c can comprise a control unit for controlling the analysis assembly and for receiving data from the analysis assembly.

By the combination of NIR/Raman/LIBS- sensors/optical systems on sugar beet pieces many of the non-sugar components can be analysed for example for monitoring the extraction efficiency and quality of the sugar beets. NIRS calibration for marc content and dry matter content shows a high performance. Based on the formula from Buchholz the monitoring of recoverable sugar on sugar beet pieces is possible, as can be seen in the following table.

| Trait | Reference | Calibration | | Validation | | | |
|---|---|---|---|---|---|---|---|
| | | min | max | min | max | R | SEP |
| Polarization | ICUMSA | 10.4 | 21.9 | 10.5 | 21.7 | 0.97 | 0.34 |
| Recoverable sugar | Calculated from polarization, Na, K, and α-amino-N (ICUMSA) | 9.0 | 20.6 | 9.5 | 20.6 | 0.95 | 0.43 |
| Dry matter | oven | 11.6 | 28.3 | 11.6 | 28.3 | 0.98 | 0.40 |
| Marc content | Wet chemistry | 2.8 | 5.1 | 3.1 | 4.9 | 0.86 | 0.20 |

NIR calibrations, based on total nitrogen- content can be used for quality estimation of sugar beets. In this case spectral information from organic nitrogen- compounds in a sugar beet (also betaine; glutamine; asparagine; pyrrolidone) are recorded in a spectral signal and included in a predicted value for nitrogen.

| Trait | Reference | Calibration | | Validation | | | |
|---|---|---|---|---|---|---|---|
| | | min | max | min | max | R | SEP |
| Total Nitrogen | Dumas | 0.48 | 1.05 | 0.5 | 1.05 | 0.77 | 0.04 |

Another option to get more information about harmful nitrogen in sugar beets is the application of LIBS sensors. High power LIBS can produce spectral signals with a higher intensity. Also the signal-to-noise ratio was increased by applying high power LIBS. For example, for the detection of nitrogen, a wavelength range from 485 - 640 nm in the spectrometer was selected. The calibration and cross-validation of total N is shown in the following table. Based on only few samples it can be summarized, that with an RPD of 2,2 a screening in low, middle and high nitrogen content is possible.

| | | **Min.** | **Max.** | **R²** | **RMSE** | **RPD** |
|---|---|---|---|---|---|---|
| **Fiber LIBS** | calibration | 11.84 | 166.65 | 0.75 | 7.73 | 2 |
| | Cross-validation | 11.84 | 166.65 | 0.63 | 9.79 | 1.6 |
| **High Power LIBS** | calibration | 11.84 | 166.65 | 0.78 | 7.22 | 2.2 |
| | Cross-validation | 11.84 | 166.65 | 0.63 | 10.08 | 1.6 |

Based on used samples it can be summarized, that with an RPD of 2,2 a screening in low, middle and high nitrogen content is possible. Based on the very small range (112-167 mg total- N/g DM) of this samples a better performance is expected by higher variation in total nitrogen content as it is common in quality estimation of sugar production facilities.

A combination of different spectroscopy methods as described above could also be applied at another analysis position, such as for example at the first analysis position or at the third analysis position.

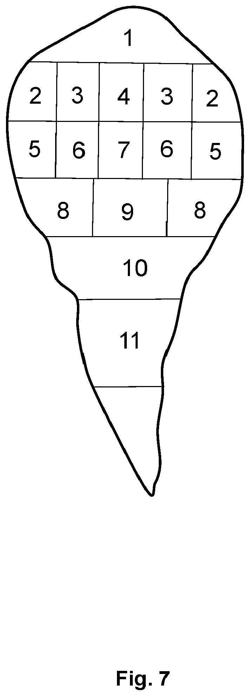

Fig. 7 shows segments of a sugar beet for investigating heterogeneity in sugar beets. The heterogeneity in sugar beets has been investigated in detail to get a better understanding of the heterogeneity of components in sugar beets. Therefore, 100 sugar beets from 2 varieties (higher tonnage variety and higher sugar content variety) were investigated. From every variety, 100 uniform beets were selected and sorted by size to process homogenous groups of 5 to 7 sugar beets. In average 16 replications per variety were produced. Every beet was divided into 6 horizontal sections. The slices were cut along specified circular vascular bundles to produce 11 segments in total. Each of the 11 segments is located at a different position of a single sugar beet, as indicated in the figure. The corresponding segments were pooled and processed by grinding and homogenization with ceramic knives in a mill. For the evaluation of the distribution of the components in the sugar beet segments different analyses were performed. The results show significant differences between segments for all components, as can be seen in the following table. For example, the concentration of recoverable sugar usually increases from the root tip to the upper root within a single sugar beet. In the central marc the content of recoverable sugar was up to 1.5 % lower compared to the root tissue outside. The highest content of recoverable sugar was found in the middle root tissue. Within sugar beets there is a significant variation for sugar content up to 2.5 %, depending on the segment. The results indicate a high heterogeneity in distribution of components within sugar beets for all analysed components.

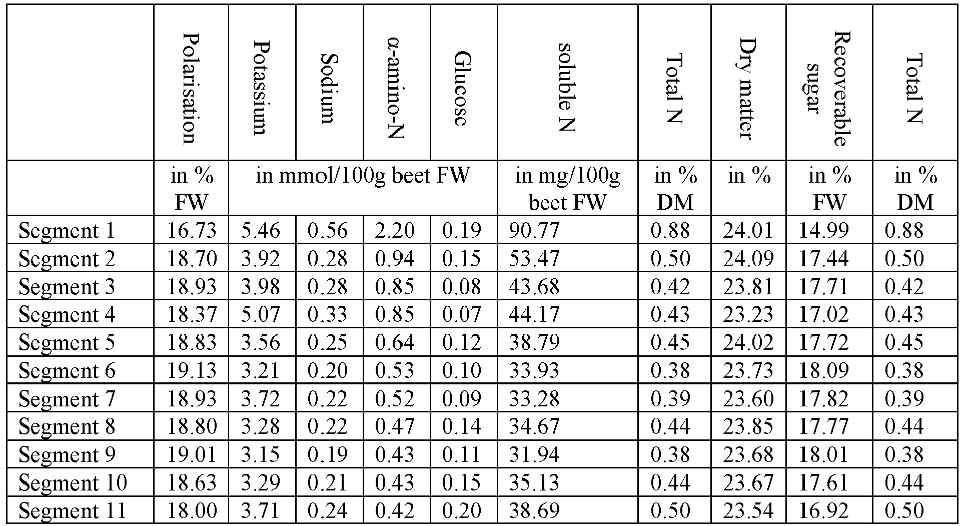

Also when comparing one sugar beet to another sugar beet the heterogeneity of components can be very high. Therefore, one truckload of sugar beet was divided in 459 individual samples. Each sample was processed and analysed in a quality lab. The results for example for recoverable sugar content show the significant heterogeneity in one grower's truckload. Recoverable sugar content ranged from 11.9 % up to 16.2 % with an average of 14.5 % and a standard deviation of 0.79 %. The standard deviation between samples is depending from sample size to evaluate the effect of sample size, random sample results were combined to virtually larger samples. The original average sample size was 11.5 sugar beets per sample. By combining two samples the standard deviation decreased from 0.79 % to 0.56 % and by merging four samples it decreased further to 0.39 %.

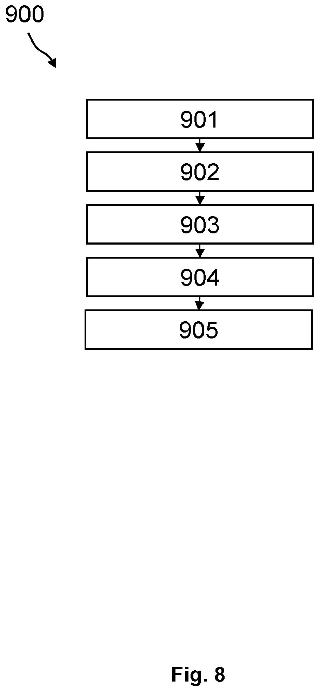

Fig. 8 shows a schematic representation of an example of a method 900 for generating calibration data for the determination of components in sugar beets for sugar production. The method 900 comprises the steps described in the following. In a step 901, taking a sample of sugar beets, preferably a determined amount, for example 10 kg. In a step 902, spectroscopic analysis of the sample by, preferably continuously, emitting electromagnetic waves towards the sample, receiving electromagnetic waves, and converting the received electromagnetic waves into a spectral signal. In a step 903, producing a sugar beet pulp from the sample, preferably by a cutter mill, and extracting the sugar beet pulp, preferably by Aluminium sulfate or lead acetate solution. In a step 904, reference analysis of the extracted sugar beet pulp by conducting measurements, such as polarimetry, flame photometry, fluorometric o-phthalaldehyde (OPA) method, copper method, immobilized enzyme biosensor method and/or others. In a step 905, comparing the results of the spectroscopic analysis with the results of the reference analysis.

### List of reference signs

- 100: method for determining components in sugar beets for sugar production
- 101: receiving a plurality of sugar beets
- 102: providing the plurality of sugar beets
- 103: analysing at least the analysis portion
- 104: emitting electromagnetic waves towards at least the analysis portion
- 105: receiving electromagnetic waves
- 106: converting the received electromagnetic waves into a spectral signal
- 107: producing sugar
- 300: sugar production facility
- 301: receiving section
- 302: truck
- 303: water source
- 304: water jet
- 305: sugar beets
- 306: beet washer
- 308: sugar beet hopper
- 310: slicing device
- 311: sugar beet pieces
- 312: transport device
- 313: roller
- 314: electromagnetic wave source
- 315, 315a, 315b, 315c: optical system
- 316, 316a, 316b, 316c: optical fiber
- 317, 317a, 317b, 317c: spectrometer
- 318, 318a, 318b, 318c: processor
- 319: sugar beet pieces mixer
- 320: diffuser
- 330: juice cleaning arrangement
- 340: filter units
- 350: evaporators
- 360: crystallization device
- 370: centrifuge
- 375: drying device
- 380: silo
- 390: mechanical press 390
- 391: pressed pulp
- 392: transport device
- 393: roller
- 394: electromagnetic wave source
- 395: optical system
- 396: optical fiber
- 397: spectrometer
- 398: processor
- D: downwards direction in diffuser
- H: hopping direction
- T: transport direction
- U: upwards direction in diffuser
- P1: first analysis position
- P2: second analysis position
- P3: third analysis position

- 400: spectral signal
- A: absorbance
- W: wavelength
- 510: first transport section
- 511: upstream end of the first transport section
- 512: downstream end of the first transport section
- 515: analysis assembly
- 520: main transport section
- 521: upstream end of the main transport section
- 522: downstream end of the main transport section
- 530: second transport section
- 531: upstream end of the second transport section
- 532: downstream end of the second transport section
- 540: bypass section
- 541: upstream end of the bypass section
- 542: downstream end of the bypass section
- 550: container
- 590: boarder of sugar production facility
- B: transport direction along the bypass section
- F: transport direction along the first transport section
- M: transport direction along the main transport section
- S: transport direction along the second transport section

- PN: polarisation measured with near-infrared spectroscopy
- PR: polarisation reference

- 900: method for generating calibration data
- 901: taking a sample of sugar beets
- 902: spectroscopic analysis of the sample
- 903: producing a sugar beet pulp
- 904: reference analysis of the extracted sugar beet pulp
- 905: comparing the results of the spectroscopic analysis with the results of the reference analysis

## Claims

1. A method (100) for determining components in industrial processing of sugar beets (305) in a production facility (300), comprising
- providing (102) a plurality of sugar beets including a production portion of sugar beets for production and an analysis portion of sugar beets for component analysis and possibly for production,
- analysing (103) at least the analysis portion and possibly the production portion, wherein analysing comprises
∘ emitting (104) electromagnetic waves towards at least the analysis portion and possibly the production portion,
∘ receiving (105) electromagnetic waves,
∘ converting (106) the received electromagnetic waves into a spectral signal (400),
- producing (107) a product from at least the production portion and possibly from the analysis portion.

2. The method according to the preceding claim
- wherein the production facility (300) is a production facility for sugar production and/or for production of animal feed and/or for biogas production and/or for ethanol production and/or for production of biodegradable plastics and/or for production of fuels and/or for production of fuel bio-components, and/or food supplement production, and/or
- wherein the production is sugar production and/or production of animal feed and/or biogas production and/or ethanol production and/or production of biodegradable plastics and/or production of fuels and/or production of fuel bio-components, and/or
- wherein the product is sugar and/or animal feed and/or biogas and/or ethanol and/or biodegradable plastics and/or fuels and/or fuel bio-components.

3. The method according to at least one of the preceding claims,
- wherein the method further comprises
∘ receiving (101) the plurality of sugar beets including the production portion of sugar beets for sugar production and the analysis portion of sugar beets for component analysis and possibly for sugar production, and/or
∘ providing sugar beet pieces (311) from the production portion and/or the analysis portion, and/or
∘ arranging at least the analysis portion and possibly the production portion onto at least one transport device (312), and/or
∘ conveying at least the analysis portion and possibly the production portion using the at least one transport device (312), preferably with a transport velocity within a range of 0.05 m/s to 20 m/s, in particular 0.05 m/s to 10 m/s, preferably within a range of 0.05 m/s to 5 m/s, more preferably within a range of 0.05 m/s to 1 m/s and/or
- wherein producing sugar comprises
∘ producing a raw juice from at least the production portion, and/or
∘ producing a thin juice, preferably from at least the raw juice, and/or
∘ producing a thick juice, preferably from at least the thin juice, and/or
∘ producing sugar, preferably from at least the thick juice, and/or
- wherein the method further comprises
∘ producing pressed pulp by removing liquid substances from at least the production portion and/or at least the analysis portion, preferably using a mechanical press.

4. The method according to at least one of the preceding claims,
- wherein analysing at least the analysis portion is conducted after the step of receiving a plurality of sugar beets, and preferably prior to storing the plurality of sugar beets, and/or
- wherein analysing at least the analysis portion is conducted after and/or during storing the plurality of sugar beets, and preferably prior to providing sugar beet pieces, in particular by slicing, and/or
- wherein analysing at least the analysis portion and possibly the production portion is conducted after providing sugar beet pieces, in particular by slicing, and preferably prior to producing a raw juice from at least the production portion, and/or
- wherein analysing at least the analysis portion and possibly the production portion is conducted after producing pressed pulp, preferably prior to and/or after drying of the pressed pulp, and/or
- wherein analysing is performed as a continuous process, preferably by emitting electromagnetic waves and/or receiving electromagnetic waves and/or converting the received electromagnetic waves into a spectral signal (400) in intervals in particular less than 100 ms, or of less than 50 ms, preferably in intervals of 10 ms, 20 ms, 30 ms or 40 ms.

5. The method according to at least one of the preceding claims,
- wherein the mass fraction of the analysis portion is at least 0.001 % or at least 0.1 % or at least 0.2 % or at least 0.5 % or at least 1 % or at least 10 % or at least 25, or at least 50 %, or at least 80 % within the plurality of sugar beets, and/or
- wherein the production portion comprises the analysis portion, and/or
- wherein the production portion and the analysis portion are, at least partly, identical.

6. The method according to at least one of the preceding claims, comprising
- generating calibration data, including
∘ taking a sample of sugar beets (305), preferably a determined amount, for example 10kg, and preferably moving the sample along a sensor that is adapted to receive electromagnetic waves,
∘ spectroscopic analysis of the sample by, preferably continuously, emitting electromagnetic waves towards the sample, receiving electromagnetic waves, and converting the received electromagnetic waves into a spectral signal (400),
∘ producing a sugar beet pulp from the sample, preferably by a cutter mill, and extracting the sugar beet pulp, preferably by Aluminium sulfate or lead acetate or water solution,
∘ reference analysis of the extracted sugar beet pulp by conducting measurements, such as polarimetry, flame photometry, fluorometric o-phthalaldehyde (OPA) method, copper method, immobilized enzyme biosensor method, oven method and/or others,
∘ comparing the results of the spectroscopic analysis with the results of the reference analysis,
- wherein preferably generating calibration data further includes one or several of the following steps:
∘ pre-processing the spectral signal for correcting and/or eliminating overlaying effects, wherein preferably pre-processing is conducted using multiplicative scatter correction (MSC), inverse MSC; extended MSC and/or derivatives, and/or derivativations, and/or smoothing, and /or standard normal variate (SNV), and/or normalization and/or combination of preprocessing methods wherein preferably pre-processing is conducted before multiple and/or multivariate and/or linear regression analysis is carried out, and/or
∘ removing spectral signals that are not converted from electromagnetic waves that are reflected from or emitted through the sugar beets, preferably by differentiating the spectral signals using classification and/or filtering, in particular using mathematical filtering methods, and/or
∘ averaging spectral signals to one spectral signal, and/or
∘ carrying out multiple and/or multivariate and/or linear regression analysis for generating calibration data, wherein preferably the calibration data is derived using principle component analysis (PCA), and/or multiple linear regression (MLR), and/or partial least squares (PLS) regression, and/or machine learning, in particular using neuronal networks.

7. The method according to at least one of the preceding claims, comprising
- processing the spectral signal for determining components in at least the analysis portion and possibly the production portion, and/or
- comparing the spectral signal with the calibration data and dependent on the comparison determine, preferably quantitatively, the components in at least the analysis portion and possibly the production portion.

8. The method according to at least one of the preceding claims,
wherein the wavelength of the electromagnetic waves lies in the infrared spectrum, preferably in the near-infrared spectrum, and/or in the microwave region and/or in the visible spectrum, and/or in the ultraviolet spectrum, and/or
wherein the spectral signal is converted by using spectroscopy, in particular near-infrared spectroscopy (NIRS), mid- infrared- spectroscopy, far-infrared spectroscopy, terahertz-spectroscopy and/or ultraviolet-visible spectroscopy (UV-Vis) and/or Raman spectroscopy and/or laser-induced breakdown spectroscopy (LIBS), and/or fluorescence spectroscopy and/or hyperspectral imaging, and/or nuclear magnetic resonance and/or a combination of hyperspectral imaging with different spectroscopic approaches and/or combinations of different spectroscopic methods,
wherein analysing is conducted using a camera and/or using a combination of a camera with different spectroscopic methods.

9. The method according to at least one of the preceding claims, comprising
- changing at least one sugar production parameter, in particular electric field pulses and/or pulse numbers and/or process temperature and/or conveying speed and/or duration of the production portion in reactor, and or and/or application of milk of lime and CO2 in raw juice purification and/or adjustment of processes of liming, carbonation, sludge separation and sulphitation in juice purification dependent on the determined components in at least the analysis portion, and/or
- changing at least one drying process parameter for drying pressed pulp, in particular drying time and/or drying temperature for drying pressed pulp dependent on the determined components in at least the analysis portion, and/or
- changing at least the order in which sugar beets are introduced into a sugar production process dependent on the determined components in at least the analysis portion.

10. The method according to at least one of the preceding claims,
wherein providing sugar beet pieces (311) from the analysis portion is conducted using a chopping device, wherein the chopping device is configured to crumble and/or cut the analysis portion into substantially equal sized sugar beet pieces, and/or
wherein providing sugar beet pieces (311) from the analysis portion and/or the production portion is conducted using a slicing device, wherein the slicing device (310) is configured to cut the analysis portion and/or the production portion into sugar beet pieces (311) that are formed as slices and/or thin elongated strips.

11. The method according to at least one of the preceding claims,
- wherein the at least one transport device comprises a first transport section (510), a main transport section (520) and a second transport section (530), wherein the main transport section is arranged downstream of the first transport section and upstream of the second transport section, and/or
- wherein the at least one transport device comprises a bypass section (540) arranged downstream of the first transport section and upstream of the second transport section, and/or
- wherein the method further comprises
∘ conveying the production portion and the analysis portion along the first transport section, and/or
∘ conveying at least the analysis portion along the bypass section, and/or
∘ conveying at least a part of the production portion, in particular the sugar beet pieces that are not part of the analysis portion, along the main transport section, and/or
∘ conveying the production portion along the second transport section, and/or
∘ conveying the analysis portion along the second transport section and/or discarding the analysis portion.

12. The method according to at least one of the preceding claims,
wherein emitting electromagnetic waves towards at least the analysis portion is conducted while the analysis portion is arranged at, and preferably conveyed along, the first transport section (510) and/or arranged at, and preferably conveyed along, the bypass section (540), and/or
wherein emitting electromagnetic waves towards the production portion is conducted while the production portion is arranged at, and preferably conveyed along, the first transport section.

13. The method according to at least one of the preceding claims, comprising
- homogeneously and/or heterogeneously distributing the sugar beet pieces onto the at least one transport device (312), preferably with a roller (313) that is arranged above the at least one transport device or without a roller.

14. The method according to at least one of the preceding claims,
- wherein the components in the analysis portion preferably comprise one or several of the following group, in particular when analysing at least the analysis portion and possibly the production portion is conducted after producing pressed pulp:
- dry matter content,
- marc content,
- ash content,
- protein content,
- soluble nitrogen compounds as proteins, betaine, amids and amino acids,
- carbohydrate content,
- crude fiber content,
- neutral detergent fiber (NDF) content,
- acid detergent fiber (ADF) content,
- lignin content,
- content of cellulose,
- content of hemicellulose,
- amino acid content,
- starch content,
- total sugar content,
- content of monosaccharides as glucose; fructose; galactose;
- content of disaccharides as sucrose, lactose, maltose;
- content of oligosaccharides as raffinose, maltodextrin, cellodextrin;
- content of polysaccharides as inulin, fructan;
- gross energy content,
- feed unit for milk production (UFL),
- metabolizable energy (ME), and/or
wherein the components in the analysis portion preferably comprise one or several of the following group, in particular when analysing at least the analysis portion and possibly the production portion is conducted after providing sugar beet pieces, in particular by slicing, and preferably prior to producing a raw juice from at least the production portion:
- total sugar content,
- content of monosaccharides as glucose; fructose; galactose;
- content of disaccharides as sucrose, lactose, maltose;
- content of oligosaccharides as raffinose, maltodextrin, cellodextrin;
- content of polysaccharides as inulin, fructas;
- extraction efficiency of sugar;
- dry matter content,
- crude protein,
- crude fiber,
- amino acids,
- starch,
- total sugar content,
- recoverable sugar content based on different formulas
- soluble nitrogen compounds as proteins, betaine, betalaine, betacyanins, betaxanthins, amids and amino acids
- phenolic substance content
- insoluble nitrogen compounds as proteins;
- nitrogen free organic substances as pectins, saponins, organic acids;
- marc content;
- fat content;
- content of alcohols;
- content of structural carbohydrates as NDF (Neutral Detergent Fiber); ADF (Acid Detergent Fiber); ADL (Acid Detergent Lignin)
- content of Hemicellulose; cellulose;
- lignin content;
- ash content;
- content of alkali metal elements and its inorganic compounds as sodium; sodium chloride;
- content of metal elements und inorganic compounds as calcium; calcium carbonate, magnesium, magnesium oxide;
- content of metalloid elements and inorganic compounds as boron, borat minerals, selenium, silicon;
- content of nonmetal elements and inorganic compounds as carbon, carbonates, phosphorus, phosphates; sulfur, iodine.

15. A method (900) for generating calibration data for the determination of components in sugar beets for sugar production, comprising
- taking (901) a sample of sugar beets, preferably a determined amount, for example 10kg, and preferably moving the sample along a sensor that is adapted to receive electromagnetic waves,
- spectroscopic analysis (902) of the sample by, preferably continuously, emitting electromagnetic waves towards the sample, receiving electromagnetic waves, and converting the received electromagnetic waves into a spectral signal (400),
- producing (903) a sugar beet pulp from the sample, preferably by a cutter mill, and extracting the sugar beet pulp, preferably by Aluminium sulfate or lead acetate solution or water,
- reference analysis (904) of the extracted sugar beet pulp by conducting measurements, such as polarimetry, flame photometry, fluorometric o-phthalaldehyde (OPA) method, copper method, immobilized enzyme biosensor method, oven method and/or others,
- comparing (905) the results of the spectroscopic analysis with the results of the reference analysis.

16. An arrangement for determining components in sugar beets for sugar production, comprising
- a receiving section (301) for receiving a plurality of sugar beets including a production portion of sugar beets for sugar production and an analysis portion of sugar beets for component analysis and possibly for sugar production,
- a chopping device, wherein the chopping device is configured to crumble and/or cut the analysis portion into substantially equal sized sugar beet pieces and/or a slicing device, wherein the slicing device (310) is configured to cut the analysis portion and/or the production portion into sugar beet pieces (311) that are formed as slices and/or thin elongated strips,
- a transport device (312) for conveying at least the analysis portion and possibly the production portion,
- an analysis assembly (515) arranged to emit electromagnetic waves towards at least the analysis portion that is arranged on the transport device, wherein preferably the analysis assembly is arranged to receive electromagnetic waves, and/or wherein preferably the analysis assembly is arranged to convert the received electromagnetic waves into a spectral signal (400),
- a raw juice production device for producing raw juice from at least the production portion, and/or
- a control unit for controlling the analysis assembly and/or for receiving data from the analysis assembly, wherein preferably the control unit is arranged for changing at least one sugar production parameter, in particular electric field pulses and/or pulse numbers and/or process temperature and/or conveying speed and/or duration of the production portion in reactor, and or and/ or application of milk of lime and CO2 in raw juice purification and/or adjustment of processes of liming, carbonation, sludge separation and sulphitation in juice purification dependent on components determined by the analysis assembly, and/or changing at least one drying process parameter for drying pressed pulp, in particular drying time and/or drying temperature for drying pressed pulp dependent on components determined by the analysis assembly, and/or changing at least the order in which sugar beets are introduced into a sugar production process dependent on components determined by the analysis assembly.

17. A sugar production facility (300) comprising an arrangement according to claim 16.

18. Use of an analysis assembly in a sugar production facility, in particular a sugar production facility according to claim 17, and/or use of an arrangement for determining components in sugar beets for sugar production, in particular an arrangement for determining components in sugar beets for sugar production according to claim 16, in a sugar production facility, in particular a sugar production facility according to claim 17, and/or use of a method for determining components in sugar beets for sugar production, in particular a method for determining components in sugar beets for sugar production according to any one of claims 1-15, in a sugar production facility, in particular a sugar production facility according to claim 17.
